(19) Europäisches Patentamt
European Patent Office
Office européen des brevets

(11) **EP 2 457 507 B1**

(12) **EUROPÄISCHE PATENTSCHRIFT**

(45) Veröffentlichungstag und Bekanntmachung des Hinweises auf die Patenterteilung:
**01.01.2020 Patentblatt 2020/01**

(51) Int Cl.:
***A61B 5/145*** *(2006.01)*

(21) Anmeldenummer: **10192472.8**

(22) Anmeldetag: **24.11.2010**

(54) **Armband mit einer Erfassungsvorrichtung zum Erfassen eines Blutbildparameters**

Arm band with a recording device for recording a blood count parameter

Bracelet doté d'un dispositif de détection pour la détection d'un hémogramme

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**30.05.2012 Patentblatt 2012/22**

(73) Patentinhaber: **eesy-innovation GmbH**
**82008 Unterhaching (DE)**

(72) Erfinder: **Fischer, Georg**
**90425, Nürnberg (DE)**

(74) Vertreter: **Bobbert, Cornelius**
**Bobbert & Partner**
**Patentanwälte PartmbB**
**Postfach 1252**
**85422 Erding (DE)**

(56) Entgegenhaltungen:
**WO-A1-2006/089763     WO-A1-2010/118537**
**DE-A1-102009 011 381     US-A1- 2005 256 411**

**Beschreibung**

Die vorliegende Erfindung betrifft das Gebiet der Erfassung einer Konzentration eines Blutbestandteiles, beispielsweise Zucker in durch ein Blutgefäß fließendem Blut.

Zur Bestimmung eines Blutbildparameters wie beispielsweise einer Konzentration eines Blutbestandteils kann invasiv Blut entnommen werden. Der Blutbildparameter kann dann unter Verwendung des entnommenen Bluts anhand standardisierter Teststreifen bestimmt werden, deren elektrische Widerstandswerte von der Konzentration des Blutbestandteiles, beispielsweise Blutzuckers, abhängen. Der jeweilige elektrische Widerstandswert kann beispielsweise unter Verwendung eines Blutzuckermessgerätes, das eine Gleichstrom-Widerstandsmessung zur Erfassung eines elektrischen Widerstandswertes eines Testreifens durchführt, erfasst werden. Der Widerstandswert kann anhand eines an sich bekannten Zusammenhanges zwischen einer Blutzuckerkonzentration und einem Widerstandswert in eine Blutzuckerkonzentration umgerechnet werden. Zur Erzielung einer hohen Erfassungsgenauigkeit ist jeder Teststreifen mit Kalibrierdaten, beispielsweise mit einem Referenzwiderstandswert oder mit einer entsprechenden Codierung, versehen, wodurch Variationen von Eigenschaften der Teststreifen ausgeglichen werden können. Nachteilig an invasiven Verfahren ist jedoch die Notwendigkeit der Blutentnahme und somit einer Verletzung eines Patienten. Außerdem ist eine kontinuierliche Erfassung einer Konzentration eines Blutbestandteils, um beispielsweise dessen Tagesgangkurve zu ermitteln, aufwändig. Darüber hinaus kann mittels des invasiven Verfahrens eine Zeitverzögerung zwischen einer Nahrungsaufnahme und beispielsweise einem Anstieg des Blutzuckers nicht genau erfasst werden. Ferner kann insbesondere bei einer geringen Konzentration des Blutzuckers im Blut ein Zeitpunkt der Insulinabgabe an den Patienten nicht genau bestimmt werden.

Zur nichtinvasiven Bestimmung eines Blutbildparameters wie beispielsweise einer Stoffkonzentration oder einer Stoffzusammensetzung im Blut können mikrowellenspektroskopische Verfahren eingesetzt werden. Die Mikrowellenspektroskopie zur Erfassung von Blutbildparametern basiert auf einer Einkopplung eines Mikrowellensignals in ein blutdurchströmtes Gewebe und einer Erfassung einer frequenzabhängigen Absorption eingekoppelter Mikrowellenenergie.

Die Druckschriften DE 10 2009 011 381 A1 betrifft eine diagnotische Messvorrichtung zur nicht-invasiven Erfassung von wenigstens einem physiologischen Parameter von Körpergewebe, mit einer optischen Messeinheit, die wenigstens eine Strahlungsquelle zur Bestrahlung des zu untersuchenden Körpergewebs und wenigstens einen Strahlungssensor zur Detektion der von dem Körpergewebe gestreuten und/oder transmittierten Strahlung umfasst. Weiter ist eine Messstrecke zur Impedanzmessung vorgesehen.

Die Druckschrift WO 2006/089763 A1 betrifft ein Verfahren sowie ein Messgerät, um die Ansorption von Licht durch lebendes, von dem Licht durchstrahltes, Gewebe bei Mensch oder Tier zu messen. Durch eine Anordnung von einem Lichtemitter und einem Lichtsensor am Armband derart, dass sie sich bei Anlegen des Armbandes auf einander gegenüberliegenden Seiten des Armquerschnittes befinden und der Emitter das Licht in den Armquerschnitt hinein einkoppelt, während der Lichtsensor mit seiner aktiven Fläche an der Oberfläche des Armes oder Handgelenkes anliegt, kann das Armband so am Arm des Benutzers fixiert werden, dass das vom Emitter ausgesandte Licht den Querschnitt des Unterarmes des benutzers durchdringt und auf diesem Wege den Sensor erreicht.

Die Druckschrift WO 2010/118537 betrifft ein Gerät zum Messen eines Parameters von lebendem Gewebe wie beispielweise einen Blutzuckerspiegel, der eine Antwort des Gewebes auf ein elektrisches Feld betrifft.

Die Druckschrift US 2005256411 A1 betrifft ein elektronisches Blutdruckmessgerät , das mithilfe eine Armbandes an einem Arm befestigt werden kann. Ein Sensor an der Innenseite eines Armbands ist jedoch nicht beschrieben.

In der Druckschrift von Andreas Caduff et al., "Non-invasive glucose monitoring in patients with Type 1 diabetes: A multi-sensor system combining sensors for dielectric and optical characterization of skin", Biosensors and Bioelectronics 24 (2009) 2778-2784, ist eine Mehrelektrodenanordnung zur mikrowellenbasierten Bestimmung eines Blutbildparameters beschrieben. Die Mehrelektrodenanordnung umfasst mehrere Elektrodenpaare mit unterschiedlichen Elektrodenabständen, durch welche unterschiedliche Eindringtiefen von Mikrowellensignalen realisiert werden können. Die Erfassung des Blutbildparameters erfolgt mittels einer Impedanzmessung, also mittels einer Eintormessung, und ist daher fehleranfällig bei etwaigen Impedanzfehlanpassungen. Aufgrund von unterschiedlichen Eindringtiefen ist mitunter keine Unterscheidung zwischen kapillarem und venösem Blut möglich, was die Messergebnisse verfälschen kann. Eine Messung eines Blutbildparameters an venösem Blut ist im Allgemeinen genauer als eine Messung des Blutbildparameters an kapillarem Blut, weil beispielsweise Blutzuckeränderungen in kapillarem Blut verzögert gegenüber venösem Blut sind.

In den Druckschriften von Buford Randal Jean et al., "A microwave frequency sensor for non-invasive blood-glucose measurement, SAS 2008 - IEEE Sensors Applications Symposium, Atlanta, GA, February 12 - 14, 2008, and M. McClung, Calibration methodology for a microwave non-invasive glucose sensor, Master Thesis, Baylor University, Mai 2008, ist eine weitere Elektrodenanordnung zur Bestimmung einer Blutzuckerkonzentration beschrieben. Dabei wird ausgenutzt, dass die dielektrischen Eigenschaften von Blut von einem Blutzuckergehalt abhängen. Durch Anpressen eines Daumens auf den Mikrowellensensor wird eine Änderung der Dielektrizitätszahl des Daumens durch eine Verstimmung eines Resonators gemessen. Durch das Anpressen des Daumes wird jedoch Blut verdrängt, was zu einer Verfälschung der Messergebnisse führen kann. Des Weiteren können die Messungen nicht kontinuierlich durchgeführt werden. Die Auswertung der Messdaten zur Bestimmung des Blutzuckergehalts hängt zudem von dem jeweiligen Patienten ab und ist daher bei anderen Patienten nicht reproduzierbar. Darüber hinaus ist mit diesem Verfahren die Eindringtiefe der Mikrowellenleistung nicht steuerbar, so dass eine Unterscheidung zwischen kapillarem und venösem Blut nicht möglich ist. Ferner wird die Änderung der Dielektrizitätszahl auf der Basis einer Eintormessung durchgeführt, welche anfällig bezüglich Fehlanpassungen ist.

Es ist die Aufgabe der vorliegenden Erfindung, ein reproduzierbares Konzept zur mikrowellenbasierten, nicht invasiven Bestimmung eines Blutbildparameters, insbesondere einer Konzentration von Blutzucker in einem durch ein Blutgefäß fließendes Blut zu schaffen.

Diese Aufgabe wird durch die Merkmale der unabhängigen Ansprüche gelöst. Vorteilhafte Weiterbildungsformen sind Gegenstand der abhängigen Ansprüche.

Die Erfindung basiert auf der Erkenntnis, dass Messungen oder Erfassungen von Blutbildparametern durch ein Armband mit einer Erfassungsvorrichtung dann reproduzierbar bereitgestellt werden können, wenn das Armband mit der Erfassungsvorrichtung mit einem vorbestimmten oder vorgegebenen Anpressdruck während der Messungen auf den Arm gedrückt wird. Zum Bereitstellen des vorgegebenen Anpressdruckes wird das Armband zusätzlich zu der Erfassungsvorrichtung mit einer Einstellvorrichtung ausgestattet. Die Einstellvorrichtung ist derart ausgebildet, dass sie den vorgegebenen oder vorbestimmten Anpressdruck zumindest während des Erfassens des Blutbildparameters durch die Erfassungsvorrichtung einstellen kann.

Das Armband mit der Erfassungsvorrichtung und der Einstellvorrichtung ist dazu eingerichtet, am Arm des Patienten, insbesondere im Bereich seines Handgelenkes, angelegt zu werden.

Dieser Ort des Handgelenkes bedingt insbesondere keine Beeinträchtigung der Bewegungsfreiheit des Patienten. Des Weiteren ist dieser Ort bei den Patienten bereits für herkömmliche Blutdruckmessgeräte etabliert. Ein weiterer Vorteil des Anlegens des Armbandes an das Handgelenk liegt darin, dass an dieser Stelle üblicherweise der Puls ertastet wird, die Haut dünn ist und somit Fehlerquellen reduziert werden können. Ferner werden durch das Anpressen des Armbandes während des Erfassens des Blutbildparameters Luftspalte vermieden, welche eine mikrowellentechnische Anpassung durch die Erfassungsvorrichtung verändern könnten. Durch das Anpressen des Armbandes mit dem vorgegebenen Anpressdruck kann sich das Armband an die Anatomie des jeweiligen Patienten anpassen.

Durch die Verwendung des Armbandes mit der Erfassungsvorrichtung und der Einstellvorrichtung kann der Blutbildparameter kontinuierlich überwacht werden. Ein Beispiel für einen solchen Blutbildparameter ist - wie oben ausgeführt - die Blutzuckerkonzentration. Durch die Möglichkeit der kontinuierlichen Überwachung der Blutzuckerkonzentration wird eine Bestimmung der Verzögerungszeit zwischen der Nahrungsaufnahme und dem Blutzuckeranstieg ermöglicht. Weiter kann auf Variationen im Tagesablauf des Patienten sehr schnell reagiert werden. Insbesondere kann bei einer Überzuckerung oder einer Unterzuckerung zeitnah ein Alarm ausgelöst werden.

Gemäß einem Aspekt der Erfindung wird ein Armband vorgeschlagen, welches eine Erfassungsvorrichtung zum Erfassen eines Blutbildparameters von Blut in einem Blutgefäß des Armes und eine Einstellvorrichtung zum Einstellen eines vorgegebenen Anpressdruckes des Armbandes auf den Arm aufweist.

Gemäß einer Ausführungsform ist die Einstellvorrichtung dazu ausgebildet, den Anpressdruck des Armbandes zumindest während des Erfassens des Blutbildparameters durch die Erfassungsvorrichtung auf den vorgegebenen Anpressdruck einzustellen.

Gemäß einer Ausführungsform hat die Einstellvorrichtung eine Sensoreinrichtung zum Messen eines aktuellen Anpressdruckes des Armbandes auf den Arm und eine Steuereinrichtung zum Einstellen des vorgegebenen Anpressdruckes in Abhängigkeit des gemessenen aktuellen Anpressdruckes.

Gemäß einer Ausführungsform ist die Sensoreinrichtung auf der Innenseite des Armbandes angeordnet.

Gemäß einer Ausführungsform ist das Armband als ein aufblasbares Armband ausgebildet.

Gemäß einer Ausführungsform weist die Einstellvorrichtung eine Luftpumpe auf, welche dazu ausgebildet ist, das Armband zum Einstellen des vorgegebenen Anpressdruckes aufzublasen.

Gemäß einer Ausführungsform umfasst die Einstellvorrichtung eine Sensoreinrichtung zum Messen eines aktuellen Anpressdruckes des Armbandes auf den Arm, eine Steuereinrichtung zum Bereitstellen eines Steuersignals in Abhängigkeit des gemessenen aktuellen Anpressdruckes und eine mittels des bereitgestellten Steuersignals gesteuerte Luftpumpe zum Aufblasen des Armbands.

Gemäß einer Ausführungsform umfasst die Erfassungsvorrichtung Elektroden, welche zur Einkopplung zumindest eines Hochfrequenzsignals in das Blutgefäß eingerichtet sind.

Gemäß einer Ausführungsform ist die Einstellvorrichtung dazu ausgebildet, den Anpressdruck der Elektroden auf den Arm auf den vorgegebenen Anpressdruck einzustellen.

Gemäß einer Ausführungsform ist die Einstellvorrichtung dazu ausgebildet, die Anpresskräfte des Armbandes während des Erfassens des Blutbildparameters durch die Erfassungsvorrichtung gleichmäßig auf den Arm zu verteilen.

Gemäß einer Ausführungsform ist die Einstellvorrichtung dazu ausgebildet, ein gleichmäßiges Anliegen des Armbandes während des Erfassens des Blutbildparameters durch die Erfassungsvorrichtung bereitzustellen.

Gemäß einer Ausführungsform ist die Einstellvorrichtung dazu ausgebildet, den Anpressdruck des Armbandes während des Erfassens des Blutbildparameters durch die Erfassungsvorrichtung konstant zu halten.

Gemäß einer Ausführungsform umfasst das Armband ferner einen Speicher zum Bereitstellen des vorgegebenen Anpressdruckes.

Vorteilhafterweise ist der in dem Speicher gespeicherte vorgegebene Anpressdruck einstellbar. Folglich können bei unterschiedlichen Patienten unterschiedliche Anpressdrücke vorgegeben werden.

Gemäß einer Ausführungsform umfasst der zumindest eine Blutbildparameter eine Glucosekonzentration in dem Blut, eine Laktatkonzentration in dem Blut oder eine Sauerstoffkonzentration in dem Blut.

Gemäß einer Ausführungsform hat die Erfassungsvorrichtung einen Sender, einen Empfänger, einen Verlustdetektor und einen Prozessor. Der Sender ist dazu eingerichtet, in das Blutgefäß ein erstes Sendesignal mit einer ersten Frequenz und ein zweites Sendesignal mit einer zweiten Frequenz einzukoppeln. Der Empfänger ist dazu eingerichtet, ein erstes Empfangssignal bei der ersten Frequenz und ein zweites Empfangssignal bei der zweiten Frequenz zu empfangen. Der Verlustdetektor ist ausgebildet, eine erste Verlustgröße auf der Basis des ersten Sendesignals und des ersten Empfangssignals zu ermitteln. Weiter ist der Verlustdetektor ausgebildet, eine zweite Verlustgröße auf der Basis des zweiten Sendesignals und des zweiten Empfangssignals zu ermitteln. Der Prozessor ist ausgebildet, eine Relaxationszeitkonstante ($\tau$) des Blutbestandteils in Abhängigkeit von der Frequenz mit der größeren Verlustgröße zu bestimmen.

Insbesondere ist der Prozessor dazu eingerichtet, die Relaxationszeitkonstante ($\tau$) des Blutbestandteils in Abhängigkeit von der ersten Frequenz zu bestimmen, wenn die erste Verlustgröße nicht kleiner als die zweite Verlustgröße ist, oder die Relaxationszeitkonstante ($\tau$) des Blutbestandteils in Abhängigkeit von der zweiten Frequenz zu bestimmen, wenn die zweite Verlustgröße nicht kleiner als die erste Verlustgröße ist.

Gemäß einer Ausführungsform hat die Erfassungsvorrichtung einen Sender, welcher ausgebildet ist, in das Blutgefäß ein erstes Sendesignal mit einer ersten Frequenz und ein zweites Sendesignal mit einer zweiten Frequenz einzukoppeln, einen Empfänger, welcher ausgebildet ist, ein erstes Empfangssignal bei der ersten Frequenz und ein zweites Empfangssignal bei der zweiten Frequenz zu empfangen, und einen Verlustdetektor, welcher ausgebildet ist, eine erste Verlustgröße auf der Basis des ersten Sendesignals und des ersten Empfangssignals bei der ersten Frequenz zu bestimmen, und eine zweite Verlustgröße auf der Basis des zweiten Sendesignals und des zweiten Empfangssignals bei der zweiten Frequenz zu bestimmen, sowie einen Prozessor, welcher ausgebildet ist, eine erste Frequenzverschiebung der ersten Verlustgröße relativ zu einer ersten Referenzverlustgröße zu bestimmen, eine zweite Frequenzverschiebung der zweiten Verlustgröße relativ zu einer zweiten Referenzverlustgröße zu bestimmen, und den Blutbildparameter auf der Basis der ersten Frequenzverschiebung und der zweiten Frequenzverschiebung zu bestimmen.

Die erste Referenzverlustgröße und die zweite Referenzverlustgröße können beispielsweise im Vorfeld anhand von Experimenten oder Messungen bestimmt werden. Handelt es sich bei der ersten Verlustgröße und der zweiten Verlustgröße beispielsweise um Absorptionslinien, so können die erste Referenzverlustgröße und die zweite Referenzverlustgröße beispielsweise Absorptionslinien einer Referenzwasserlösung mit einer vorbestimmten Konzentration eines Blutbestandteils, beispielsweise Blutzuckers, sein.

Gemäß einer Ausführungsform betrifft die Erfindung eine Erfassungsvorrichtung zum Erfassen eines Blutbildparameters von Blut in einem Blutgefäß, mit einem Sender mit einer Anzahl von Sendeantennen zum Aussenden von zumindest einem Sendesignal, einem Empfänger mit einer Anzahl von Empfangsantennen zum Empfangen von zumindest einem Empfangssignal, einem Prozessor, welcher ausgebildet ist, eine erste Erfassungskonfiguration umfassend eine Sendeantenne der Anzahl der Sendeantennen und eine Empfangsantenne der Anzahl der Empfangsantennen auszuwählen, und eine zweite Erfassungskonfiguration umfassend eine Sendeantenne der Anzahl der Sendeantennen und eine Empfangsantenne der Anzahl von Empfangsantennen auszuwählen, einem Verlustdetektor, welcher ausgebildet ist, bei Auswahl der ersten Erfassungskonfiguration zum Aussenden eines Sendesignals eine erste Verlustgröße auf der Basis des Sendesignals und eines Empfangssignals zu erfassen, und bei Auswahl der zweiten Erfassungskonfiguration zum Aussenden eines Sendesignals eine zweite Verlustgröße auf der Basis des Sendesignals und eines Empfangssignals zu erfassen, wobei der Prozessor ausgebildet ist, die Erfassungskonfiguration mit der geringeren Verlustgröße zum Erfassen des Blutbildparameters auszuwählen.

Bevorzugt wird bei der Auswahl der jeweiligen Erfassungskonfiguration das Blutgefäß erregt, wobei die Sendesignale beispielsweise in Richtung des Blutgefäßes ausgesendet werden. Auf der Basis der Empfangssignale, welche empfangene Versionen der Sendesignale sind, sowie auf der Basis der Sendesignale kann beispielsweise dasjenige Antennenpärchen umfassend eine Sendeantenne und eine Empfangsantenne als diejenige Erfassungskonfiguration ausgewählt werden, die mit den geringsten Einkoppelverlusten verbunden ist. Die Einkoppelverluste können beispielsweise auf der Basis eines Vergleichs der vorgenannten Verlustgrößen, beispielsweise Absorptionslinien oder Dämpfungen, erfasst werden.

Ebenso offenbart ist ein Verfahren zum Betreiben eines Armbandes mit einer Erfassungsvorrichtung zum Erfassen eines Blutbildparameters von Blut in einem Blutgefäß des Armes, wobei ein vorgegebener Anpressdruck des Armbandes auf den Arm eingestellt wird.

Weitere Ausführungsbeispiele werden Bezug nehmend auf die beiliegenden Zeichnungen näher erläutert. Es zeigen:

Fig. 1　　　ein schematisches Blockdiagramm eines Armbandes;

Fig. 2　　　ein schematisches Blockdiagramm eines Ausschnittes eines Armbandes;

Fig. 3　　　ein schematisches Blockdiagramm eines Ausschnittes eines Armbandes;

Fig. 4　　　ein schematisches Blockdiagramm einer Anordnung der Elektroden der Erfassungsvorrichtung;

Fig. 5　　　ein schematisches Ablaufdiagramm eines Verfahrens zum Betreiben eines Armbandes;

Fig. 6　　　ein schematisches Blockdiagramm eines Ausführungsbeispiels einerErfassungsvorrichtung;

Fig. 7　　　ein Diagramm zur Illustrierung der reellen Dielektrizitätszahl $\varepsilon'$ und der komplexen Dielektrizitätszahl $\varepsilon''$ in Abhängigkeit der Frequenz;

Fig. 8　　　ein Diagramm zur Illustrierung eines Zusammenhangs zwischen der Relaxationszeitkonstanten $(\tau)$ und der Glucosekonzentration im Blut;

Fig. 9　　　ein schematisches Blockdiagramm eines Ausführungsbeispiels einer Erfassungsvorrichtung mit einer Kommunikationsvorrichtung;

Fig. 10　　　ein schematisches Ablaufdiagramm eines Ausführungsbeispiels eines Verfahrens zum Erfassen eines Blutbildparameters von Blut in einem Blutgefäß;

Fig. 11　　　ein Blockdiagramm einer Erfassungsvorrichtung;

Fig. 12　　　ein Modell eines Querschnitts eines menschlichen Unterarms;

Fig. 13A-13D　Antennen;

Fig. 14　　　eine elektrische Dipolantenne;

Fig. 14B　　　eine Erregeranordnung;

Fig. 15A, 15B　Erregeranordnungen;

Fig. 16A　　　eine Schleifenantenne;

Fig. 16B　　　eine Erregeranordnung;

Fig. 17　　　eine Erregeranordnung;

Fig. 18　　　eine Erregeranordnung;

Fig. 19　　　eine Erregeranordnung;

Fig. 20　　　eine Erregeranordnung;

Fig. 21　　　ein Blockdiagramm einer Erfassungsvorrichtung;

Fig. 22　　　eine Frequenzverschiebung eines Absorptionsmaximums;

Fig. 23　　　ein Transmissionsverhalten;

Fig. 24　　　Frequenzverschiebungen;

Fig. 25　　　ein Diagramm eines Verfahrens zum Erfassen eines Blutbildparameters, und

Fig. 26　　　ein Blockdiagramm einer Erfassungsvorrichtung.

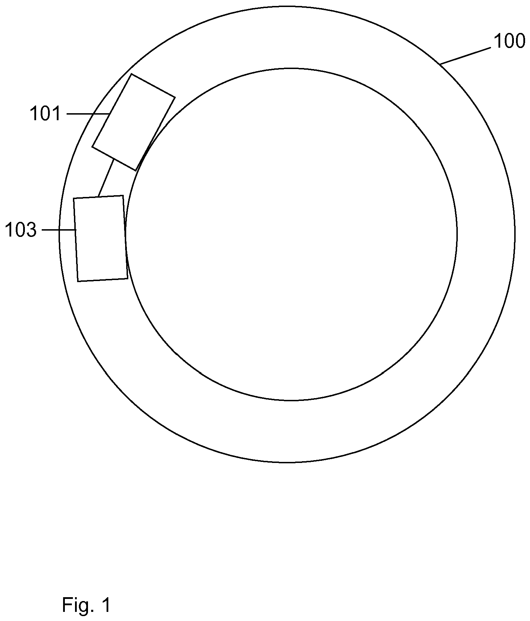

Die Fig. 1 zeigt ein Blockdiagramm eines Ausführungsbeispiels eines Armbandes 100 mit einer Erfassungsvorrichtung 101 und einer Einstellvorrichtung 103. Die Erfassungsvorrichtung 101 ist zum Erfassen eines Blutbildparameters von Blut in einem Blutgefäß des Armes eingerichtet. Ein Beispiel für den zu erfassenden Blutbildparameter ist die Glukosekonzentration in dem Blut.

Die Einstellvorrichtung 103 ist zum Einstellen eines vorgebbaren Anpressdruckes des Armbandes 100 auf den Arm eingerichtet. Durch Einstellen des vorgegebenen Anpressdruckes des Armbandes 100 kann die Einstellvorrichtung 103 reproduzierbare Erfassungen des Blutbildparameters durch die Erfassungsvorrichtung 101 sicherstellen. Dazu ist die Einstellvorrichtung 103 insbesondere dazu eingerichtet, den Anpressdruck des Armbandes 100 während des Erfassens des Blutbildparameters durch die Erfassungsvorrichtung 101 auf den vorgebbaren Anpressdruck einzustellen.

Das Armband 100 ist insbesondere als ein aufblasbares Armband 100 ausgebildet. Dabei hat die Einstellvorrichtung 103 insbesondere eine Luftpumpe, welche dazu ausgebildet ist, das Armband 100 zum Einstellen des vorgegebenen Anpressdruckes aufzublasen.

Im Detail umfasst die Erfassungsvorrichtung 101 insbesondere Elektroden, welche zur Einkopplung zumindest eines Hochfrequenzsignals in das Blutgefäß eingerichtet sind. Das Hochfrequenzsignal ist dazu ausgebildet, einen Parameter zur Erfassung des Blutbildparameters zu liefern. Ein Beispiel für einen solchen Parameter bildet die Relaxationszeitkonstante $(\tau)$ des Blutbildparameters. Dabei ist die Einstellvorrichtung 103 insbesondere dazu ausgebildet, den Anpressdruck der Elektroden auf den Arm auf den vorgegebenen Anpressdruck einzustellen.

Des Weiteren kann die Einstellvorrichtung 103 derart ausgebildet werden, dass sie die Anpresskräfte des Armbandes 100 während des Erfassens des Blutbildparameters durch die Erfassungsvorrichtung 101 gleichmäßig auf den Arm verteilt. Ferner ist die Einstellvorrichtung 103 vorzugsweise derart ausgebildet, dass sie ein gleichmäßiges Anliegen des Armbandes 100 während des Erfassens des Blutbildparameters durch die Erfassungsvorrichtung 101 sicherstellt.

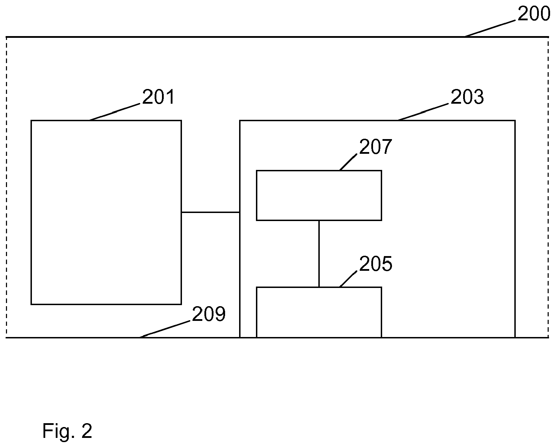

Die Fig. 2 zeigt ein Blockschaltbild eines Ausschnittes eines Ausführungsbeispiels eines Armbandes 200. Das Armband 200 hat eine Erfassungsvorrichtung 201 und eine Einstellvorrichtung 203. Die Erfassungsvorrichtung 201 und die Einstellvorrichtung 203 sind zumindest wie die Erfassungsvorrichtung 101 und die Einstellvorrichtung 103 der Fig. 1 ausgebildet. Des Weiteren hat die Einstellvorrichtung 203 der Fig. 2 eine Sensoreinrichtung 205 und eine Steuereinrichtung 207. Die Sensoreinrichtung 205 ist dazu eingerichtet, einen aktuellen Anpressdruck des Armbandes 200 auf den Arm zu messen. In Abhängigkeit des gemessenen aktuellen Anpressdruckes stellt die Steuereinrichtung 207 den vorgegebenen Anpressdruck auf den Arm ein.

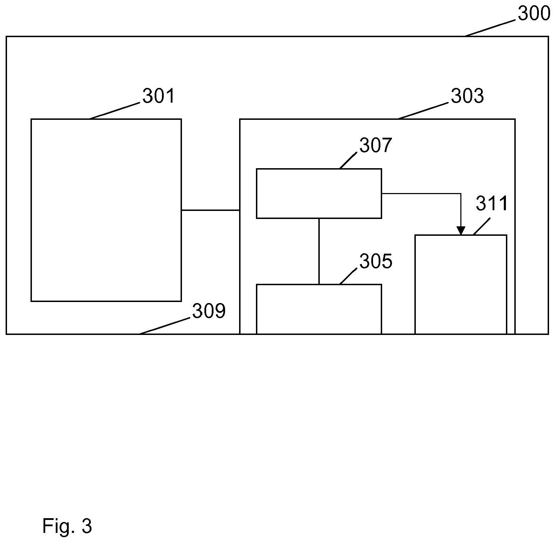

Die Fig. 3 zeigt ein Blockschaltbild eines Ausschnittes eines weiteren Ausführungsbeispiels eines Armbandes 300. Das Armband 300 hat eine Erfassungsvorrichtung 301 und eine Einstellvorrichtung 303. Die Einstellvorrichtung 303 hat eine Sensoreinrichtung 305, eine Steuereinrichtung 307 und eine Luftpumpe 311. Die Sensoreinrichtung 305 misst einen aktuellen Anpressdruck des Armbandes 300 auf den Arm. Die Steuereinrichtung 307 stellt ein Steuersignal in Abhängigkeit des gemessenen aktuellen Anpressdruckes bereit. Mittels des bereitgestellten Steuersignals wird die Luftpumpe 311 zum Aufblasen des Armbandes 300 gesteuert.

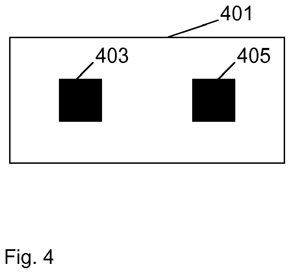

In Fig. 4 ist ein schematisches Blockdiagramm einer Anordnung 400 der Elektroden 403, 405 der Erfassungsvorrichtung zum Erfassen eines Blutbildparameters von Blut in einem Blutgefäß des Armes dargestellt.

Ohne Einschränkung der Allgemeinheit zeigt die Anordnung 400 nur zwei Elektroden 403 und 405. Die Anordnung 400 ist insbesondere Teil der Erfassungsvorrichtung und beispielsweise als eine Platte mit beispielhaften Abmessungen von 5 cm auf 2 cm ausgestaltet. Die Elektroden 403, 405 haben beispielsweise eine Grundfläche von 5 mm auf 5 mm. Der Abstand der Elektroden 403, 405 beträgt beispielsweise 1 bis 2 cm. Damit wird zum einen eine genügend starke Transmission erzielt und zum anderen wird eine genügend große Eindringtiefe in den Körper sichergestellt.

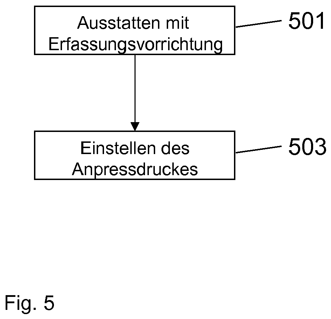

Die Fig. 5 zeigt ein schematisches Ablaufdiagramm eines Verfahrens zum Betreiben eines Armbandes mit einer Erfassungsvorrichtung.

In Schritt 501 wird das Armband mit einer Erfassungsvorrichtung zum Erfassen eines Blutbildparameters von Blut in einem Blutgefäß des Armes ausgestattet. Die Erfassungsvorrichtung ist beispielsweise gemäß einem der Ausführungsbeispiele der Figuren 1, 2 oder 3 ausgebildet.

In Schritt 503 wird ein vorgegebener Anpressdruck des Armbandes auf den Arm eingestellt. Somit wird ein reproduzierbares Erfassen des Blutbildparameters durch die Erfassungsvorrichtung sichergestellt.

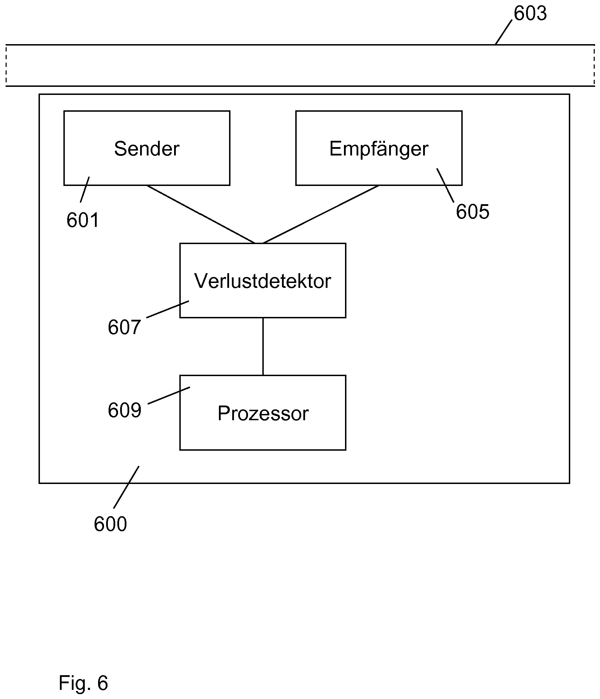

Fig. 6 zeigt ein Blockdiagramm einer Erfassungsvorrichtung 600 zum Erfassen eines Blutbildparameters, wie beispielsweise einer Konzentration von Blutzucker oder Glucose. Die Erfassungsvorrichtung 600 umfasst einen Sender 601, welcher ausgebildet ist, in das in Fig. 6 schematisch dargestellte Blutgefäß 603 ein erstes Sendesignal mit einer ersten Frequenz und ein zweites Sendesignal mit einer zweiten Frequenz einzukoppeln. Das erste Sendesignal und das zweite Sendesignal können beispielsweise gemeinsam ein Breitbandsignal ergeben. Der Sender 601 kann weiter dazu ausgebildet sein, das erste Sendesignal und das zweite Sendesignal sequenziell hintereinander in das Blutgefäß 603 einzukoppeln. Hierzu kann der Sender 601 eine oder mehrere Sendeantennen aufweisen, welche beispielsweise als Dipolantennen ausgebildet sind.

Die Erfassungsvorrichtung 600 umfasst ferner einen Empfänger 605, welcher ausgebildet ist, ein erstes Empfangssignal bei der ersten Frequenz und ein zweites Empfangssignal bei der zweiten Frequenz zu empfangen. Hierzu kann der Empfänger 605 eine oder mehrere Empfangsantennen aufweisen.

Des Weiteren hat die Erfassungsvorrichtung 600 einen Verlustdetektor 607, welcher beispielsweise mit dem Sender 601 und dem Empfänger 605 gekoppelt ist und dazu vorgesehen ist, eine erste Verlustgröße auf der Basis des ersten Sendesignals und des ersten Empfangssignals sowie eine zweite Verlustgröße auf der Basis des zweiten Sendesignals und des zweiten Empfangssignals zu bestimmen.

Die Erfassungsvorrichtung 600 weist ferner einen Prozessor 609 auf, welcher mit dem Verlustdetektor 607 gekoppelt ist und dazu vorgesehen ist, eine Relaxationszeitkonstante ($\tau$) des Blutbildparameters in Abhängigkeit von der Frequenz mit der größeren Verlustgröße zu bestimmen.

Beispielsweise wird der Prozessor 609 die Relaxationszeitkonstante ($\tau$) des Blutbildparameters in Abhängigkeit von der ersten Frequenz bestimmen, wenn die erste Verlustgröße größer als die zweite Verlustgröße ist. Entsprechend wird der Prozessor 609 die Relaxationszeitkonstante ($\tau$) des Blutbildparameters in Abhängigkeit von der zweiten Frequenz bestimmen, wenn die zweite Verlustgröße größer als die erste Verlustgröße ist.

Die in Fig. 6 dargestellte Erfassungsvorrichtung 600 nutzt die Erkenntnis aus, dass ein Blutgefäß, wie beispielsweise eine Vene, eine Hautschicht bzw. eine Vene umgebendes Fettgewebe als ein dielektrischer Wellenleiter aufgefasst werden kann. Der Aufbau eines menschlichen Unterarms ist in Netter, F.N., Atlas der Anatomie, Thieme-Verlag, 2006, beschrieben. Demnach besteht ein menschlicher Unterarm im Querschnitt aus zwei Knochen, welche von einem Muskelgewebe umgeben sind. Um das Muskelgewebe herum sind oberflächliche Venen verteilt. Die Knochen, das Muskelgewebe sowie die Venen sind durch ein Fettgewebe ummantelt, welches durch obere Hautschichten bedeckt ist. Die oberflächlichen Venen sind relativ nahe an den oberen Hautschichten angeordnet und von diesen durch das Fettgewebe separiert.

Werden beispielsweise der in Fig. 6 dargestellte Sender 601 und der Empfänger 605 auf die obere Hautschicht aufgelegt, so kann der Sender 601 dazu eingesetzt werden, in den durch eine Vene, ein Fettgewebe sowie eine Hautschicht gebildeten dielektrischen Wellenleiter eine transversal-elektrische (TE) oder eine transversal-magnetische (TM) Welle einzukoppeln. Dabei kann die Hautschicht sowie das Fettgewebe als ein Filmwellenleiter verstanden werden.

Wie oben bereits ausgeführt, ist der Verlustdetektor 607 dazu eingerichtet, eine erste Verlustgröße auf der Basis des ersten Sendesignals und des ersten Empfangssignals zu ermitteln und eine zweite Verlustgröße auf der Basis des zweiten Sendesignals und des zweiten Empfangssignals zu ermitteln. Bei Verwendung weiterer Sendesignal- und Empfangssignalpaare wird der Verlustdetektor 607 entsprechend weitere Verlustgrößen ermitteln.

Insbesondere ist der Verlustdetektor 607 dazu ausgebildet, die Verlustgrößen durch eine Zweitormessung zu bestimmen. Der Verlustdetektor 607 umfasst beispielsweise einen Netzwerkanalysator oder einen Leistungsdetektor.

Des Weiteren ist der Verlustdetektor 607 dazu eingerichtet, zur Bestimmung der Verlustgrößen jeweils einen Vorwärtstransmissionsfaktor $S_{21}$ und einen Eingangsreflexionsfaktor $S_{11}$ zu bestimmen.

Dabei wird der Verlustdetektor die jeweilige Verlustgröße Pveriust mittels folgender Formel berechnen:

$$P_{Verlust} = 1 - |S_{11}|^2 - |S_{21}|^2.$$

Insbesondere ist der Verlustdetektor 607 dazu eingerichtet, zur Bestimmung der jeweiligen Verlustgröße die komplexe Dielektrizitätszahl $\varepsilon''$ zu ermitteln.

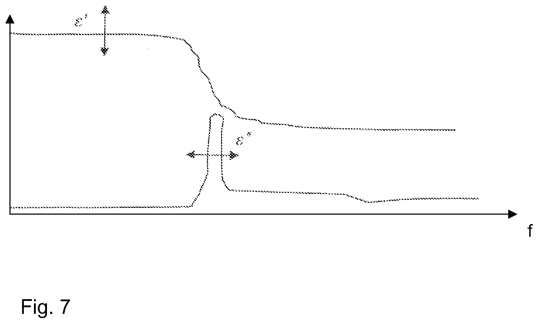

Hierzu zeigt die Fig. 7 ein Diagramm zur Illustrierung der reellen Dielektrizitätszahl $\varepsilon'$ und der komplexen Dielektrizitätszahl $\varepsilon''$ in Abhängigkeit der Frequenz f.

Dabei illustriert die Fig. 7, dass in dem Frequenzbereich, wo der Realteil $\varepsilon'$ von dem höheren Niveau auf das niedrigere Niveau übergeht, die durch die komplexe Dielektrizitätszahl $\varepsilon''$ repräsentierten Verluste steigen. Dieser Anstieg der Verluste wird in der Spektroskopie auch als Absorptionslinien bezeichnet. Der Effekt, der hierbei ausgenützt werden kann, ist der, dass sich die Frequenz, bei der die Überhöhung der Verluste - siehe lokales Maximum von $\varepsilon''$ - mit der Konzentration des Zuckergehaltes verschiebt.

Beispielsweise der menschliche Körper besteht zu 80 % aus Wasser. Wasser besitzt Absorptionslinien, z. B. bei 19 GHz und 50 GHz. Deren Verstimmung kann bestimmt werden und auf den Zuckergehalt abgebildet werden. Die Verstimmung der Resonanzfrequenz bei $\varepsilon''$ ist - wie Fig. 7 illustriert - leichter detektierbar als die Plateauveränderung von $\varepsilon'$. Insbesondere verschieben Variationen in der Ankopplung die Frequenz des Maximums von $\varepsilon''$ vorteilhafterweise nicht. Damit ist eine Bestimmung der Zuckerkonzentration aus der Beobachtung von $\varepsilon''$ deutlich weniger fehleranfällig als die Beobachtung von $\varepsilon'$ bzw. dessen Niveauänderungen.

Weil bei einer Vielzahl von Stoffen solche Kurven wie die der Fig. 7 überlagert sind, ist eine Separation der Stoffe durch eine Beobachtung der imaginären Dielektrizitätszahl $\varepsilon''$ einfacher durchführbar, da jedem Stoff ein bestimmtes Absorptionsmaximum zugeordnet werden kann. Bei der reellen Dielektriziätszahl $\varepsilon'$ kann jedoch nur die Summe aller reellen Dielektrizitätszahlen $\varepsilon'$ aller beteiligten Stoffe beobachtet werden.

Wie oben bereits ausgeführt, ist der Prozessor 609 dazu ausgebildet, die Relaxationszeitkonstante i des Blutbildparameters in Abhängigkeit von der Frequenz mit der größeren oder maximalen Verlustgröße zu bestimmen. Weiter ist der Prozessor 609 dazu ausgebildet, den Blutbildparameter, wie die Glucosekonzentration im Blut, in Abhängigkeit der bestimmten Relaxationszeitkonstanten zu ermitteln.

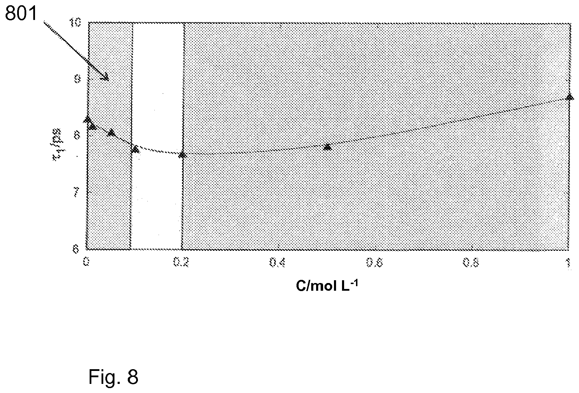

Dazu zeigt die Fig. 8 ein Diagramm zur Illustrierung eines Zusammenhangs zwischen der Relaxationszeitkonstanten ($\tau$) und der Glucosekonzentration C/mol L$^{-1}$ im Blut. Dabei zeigt der mit dem Bezugszeichen 801 referenzierte Bereich in der Fig. 8 einen kritischen Blutzuckerbereich.

Ferner ist der Prozessor 609 insbesondere dazu ausgebildet, die Relaxationszeitkonstante ($\tau$) auf der

Basis der Formel $\tau = \dfrac{1}{2\pi f_A}$ zu berechnen, wobei $f_A$ die Frequenz bezeichnet, bei welcher die ermittelte Verlustgröße maximal ist.

Vorteilhafterweise ist dann der Prozessor 609 dazu ausgebildet, die Frequenz zu bestimmen, bei welcher der Imaginärteil der komplexen Dielektrizitätszahl $\varepsilon''$ maximal ist, und die Relaxationszeitkonstante ($\tau$) in Abhängigkeit der bestimmten Frequenz zu ermitteln ist. Diese bestimmte Frequenz nutzt der Prozessor 609 dann zur Bestimmung des Blutbildparameters, wie der Glucosekonzentration.

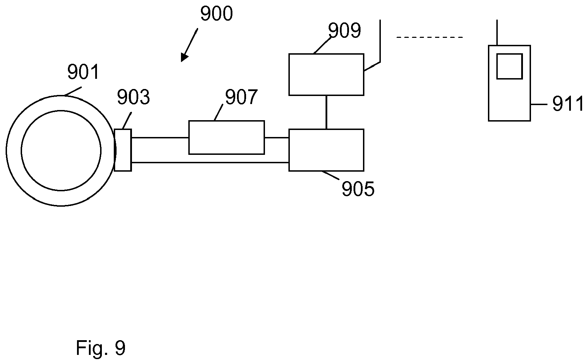

Die Fig. 9 zeigt ein schematisches Blockdiagramm einer Erfassungsvorrichtung 900. Die Erfassungsvorrichtung 900 hat ein Armband 901, einen an dem Armband 901 befestigten Sensor-Array 903, einen Mikroprozessor 905, einen Mikrowellenschaltkreis 907 zur Generierung der Sendesignale und eine Kommunikationsvorrichtung 909.

Der Sensor-Array 903 hat beispielsweise einen Mikrowellensensor, einen Temperatursensor und einen Feuchtigkeitssensor.

Der Mikroprozessor 905 ist beispielsweise wie der Prozessor 609 der Fig. 6 ausgebildet.

Die Kommunikationsvorrichtung 909 ist dazu eingerichtet, eine Kommunikationsverbindung für die Erfassungsvorrichtung 900 zu einer weiteren Kommunikationsvorrichtung 911 bereitzustellen. Die Kommunikationsvorrichtung 909 umfasst beispielsweise eine Bluetooth-Schnittstelle. Die weitere Kommunikationsvorrichtung 911 ist beispielsweise ein Mobilfunkgerät, ein Smartphone oder eine GPS-basierte Einrichtung.

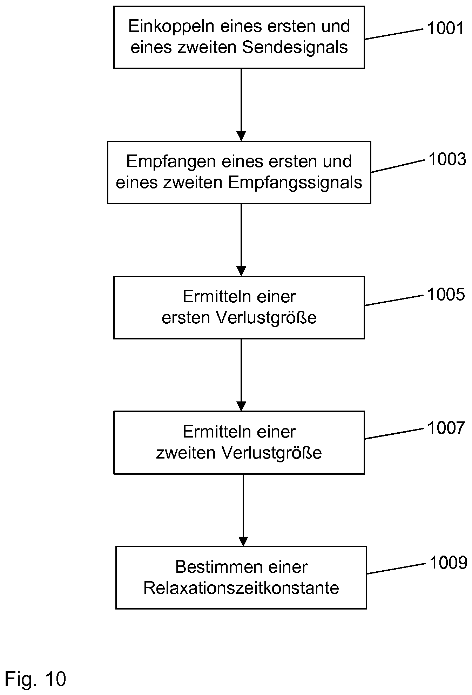

In Fig. 10 ist ein schematisches Ablaufdiagramm eines Ausführungsbeispiels eines Verfahrens zum Erfassen eines Blutbildparameters, wie beispielsweise einer Glucosekonzentration, von Blut in einem Blutgefäß dargestellt.

In Schritt 1001 werden ein erstes Sendesignal mit einer ersten Frequenz und ein zweites Sendesignal mit einer zweiten Frequenz in das Blutgefäß eingekoppelt.

In Schritt 1003 werden ein erstes Empfangssignal bei der ersten Frequenz und ein zweites Empfangssignal bei der zweiten Frequenz empfangen.

In Schritt 1005 wird eine erste Verlustgröße auf der Basis des ersten Sendesignals und des ersten Empfangssignals ermittelt.

In Schritt 1007 wird eine zweite Verlustgröße auf der Basis des zweiten Sendesignals und des zweiten Empfangssignals ermittelt.

In Schritt 1009 wird eine Relaxationszeitkonstante des Blutbildparameters in Abhängigkeit von der Frequenz mit der größeren Verlustgröße bestimmt. Abhängig von der bestimmten Relaxationszeitkonstanten kann dann beispielsweise die Glucosekonzentration in dem Blut bestimmt werden.

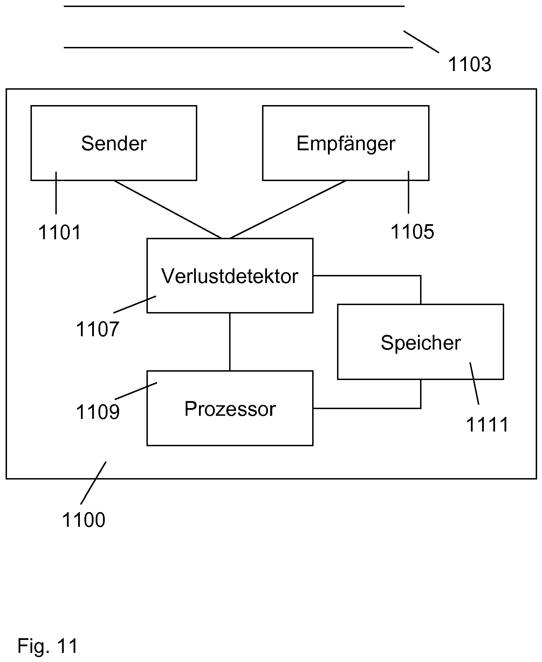

Fig. 11 zeigt ein Blockdiagramm einer Erfassungsvorrichtung 1100 zum Erfassen eines Blutbildparameters wie beispielsweise einer Konzentration von Blutzucker. Die Erfassungsvorrichtung 1100 umfasst einen Sender 1101, welcher ausgebildet ist, in das in Fig. 11 schematisch dargestellte Blutgefäß 1103 ein erstes Sendesignal mit einer ersten Frequenz und ein zweites Sendesignal mit einer zweiten Frequenz einzukoppeln. Das erste Sendesignal und das zweite Sendesignal können beispielsweise gemeinsam ein Breitbandsignal ergeben. Der Sender 1101 kann ausgebildet sein, das erste Sendesignal und das zweite Sendesignal hintereinander, beispielsweise durch einen Frequenz-Sweep, auszusenden. Hierzu kann der Sender 1101 eine oder mehrere Sendeantennen, welche beispielsweise als Dipolantennen oder Rahmenantennen oder Patch-Antennen ausgebildet sein können, aufweisen.

Die Erfassungsvorrichtung 1100 umfasst ferner einen Empfänger 1105, welcher ausgebildet ist, ein erstes Empfangssignal bei der ersten Frequenz und ein zweites Empfangssignal bei der zweiten Frequenz zu empfangen. Hierzu kann der Empfänger 1105 eine oder mehrere Empfangsantennen aufweisen.

Die Erfassungsvorrichtung 1100 umfasst ferner einen Verlustdetektor 1107, welcher beispielsweise mit dem Sender 1101 und dem Empfänger 1105 gekoppelt und vorgesehen ist, eine erste Verlustgröße auf der Basis des ersten Sendesignals und des ersten Empfangssignals sowie eine zweite Verlustgröße auf der Basis des zweiten Sendesignals und des zweiten Empfangssignals zu bestimmen.

Die Erfassungsvorrichtung umfasst ferner einen Prozessor 1109, welcher mit dem Verlustdetektor 1107 gekoppelt und vorgesehen ist, eine erste Frequenzverschiebung der ersten Verlustgröße relativ zu einer ersten Referenzverlustgröße und eine zweite Frequenzverschiebung der zweiten Verlustgröße relativ zu einer zweiten Referenzverlustgröße zu bestimmen. Der Prozessor 1109 kann ferner ausgebildet sein, den Blutbildparameter auf der Basis der beiden Frequenzverschiebungen zu bestimmen.

Die Erfassungsvorrichtung 1100 kann ferner einen Speicher 1111 aufweisen, auf welchen beispielsweise der Prozessor 1109 und optional der Verlustdetektor 1107 zugreifen können. In dem Speicher 1111 sind beispielsweise die erste und die zweite Referenzverlustgröße oder eine Mehrzahl von Referenzverlustgrößen abgelegt. Bei den Referenzverlustgrößen kann es sich beispielsweise um Absorptionen oder Absorptionslinien einer Wasserlösung mit einem Blutbestandteil, beispielsweise Blutzucker, handeln. Die auf der Basis der Frequenzverschiebungen erfassten Verlustgrößen können frequenzverschobene Absorptionen oder Absorptionslinien sein, so dass auf der Basis der Frequenzverschiebungen der Blutbildparameter, wie beispielsweise eine Konzentration von Blutzucker, ermittelt werden kann.

Die in Fig. 11 dargestellte Erfassungsvorrichtung 1100 nutzt die Erkenntnis aus, dass ein Blutgefäß, eine Hautschicht sowie ein das Blutgefäß umgebendes Fettgewebe von beispielsweise einem menschlichen Unterarm als ein dielektrisches Wellenleitersystem aufgefasst werden können. Werden beispielsweise der in Fig. 11 dargestellte Sender 1101 und der Empfänger 1105 auf die obere Hautschicht aufgelegt, so kann der Sender 1101 dazu eingesetzt werden, in das durch ein Blutgefäß, ein Fettgewebe sowie eine Hautschicht gebildete dielektrische Wellenleitersystem beispielsweise eine transversalelektrische (TE) Welle oder eine transversal-magnetische (TM) Welle einzukoppeln. Dabei kann die Hautschicht sowie das Fettgewebe als ein Filmwellenleiter verstanden werden.

Wird nun beispielsweise ein Mikrowellenmesskopf, wie er für die Bestimmung einer komplexen Dielektrizitätszahl von Materialien benutzt werden kann, verwendet, so kann dadurch das Stoffgemisch bestehend aus Haut, Fettgewebe und Venen charakterisiert werden.

Um einen Blutbildparameter zu erfassen, ist es vorteilhaft, im Wesentlichen nur das venöse Blut zu erfassen. Hierzu kann der Sender 1101 ausgebildet sein, das Sendesignal in der Gestalt einer elektromagnetischen Welle direkt in das Blutgefäß einzukoppeln. Der Sender 1101 sowie der Empfänger 1105 können jeweils eine Mehrzahl von Antennen aufweisen, so dass zur Einkopplung der elektromagnetischen Welle in das Blutgefäß und zur Auskopplung einer elektromagnetischen Welle aus dem Blutgefäß jeweils diejenige Sendeantenne und diejenige Empfangsantenne ausgewählt werden können, welche mit den geringsten Koppelverlusten verbunden sind.

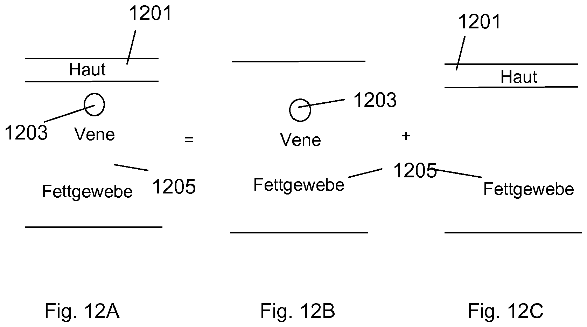

In den Figuren 12A bis 12C ist ein vereinfachtes Modell eines Querschnitts eines menschlichen Unterarms, z. B. eines Handgelenks, wie es beispielsweise für Feldsimulationen bzw. zur Modellierung eines dielektrischen Wellenleitersystems benutzt werden kann. Wie in Fig. 12A dargestellt, umfasst das Modell eine Hautschicht 1201, ein Blutgefäß 1203 sowie ein das Blutgefäß 1203 umgebendes Fettgewebe 1205. Das in Fig. 12A dargestellte Modell bildet ein dielektrisches Wellenleitersystem umfassend den in Fig. 12B dargestellten dielektrischen Wellenleiter sowie den in Fig. 12C dargestellten elektrischen Filmwellenleiter.

Der in Fig. 12B dargestellte dielektrische Wellenleiter umfasst das Blutgefäß 1203 sowie das dieses umgebende Fettgewebe 1205. Der dielektrische Filmwellenleiter aus Fig. 12C umfasst hingegen die Hautschicht 1201 sowie das Fettgewebe 1205. Für die Hautschicht 1201, das Fettgewebe 1205 sowie für das Blutgefäß 1203 kann jeweils ein unterschiedliches dispersives, d. h. frequenzabhängiges, Verhalten der jeweiligen komplexen Dielektrizitätszahl angesetzt werden. Das oben liegende Blutgefäß 1203 wird dabei als ein dielektrischer Wellenleiter interpretiert, in dem je nach Frequenz verschiedene Moden bzw. Wellentypen, beispielsweise eine TE-Welle, eine TM-Welle, eine TEM-Welle oder eine HE-Welle, ausbreitungsfähig sein können. Zu dem Wellenleitermechanismus in dem dielektrischen Wellenleiter kommt ein weitererWellenleitermechanismus in der Gestalt des in Fig. 12C dargestellten Filmwellenleiters hinzu, der durch die obere Hautschicht 1201 gebildet wird.

Eine Sendeantenne des Senders 1101 sowie eine Empfangsantenne des Empfängers 1105 können bevorzugt derart ausgestaltet sein, dass sie dezidiert Mikrowellenleistung in das Blutgefäß 1203 einkoppeln und diese beispielsweise nach einigen Zentimetern wieder auskoppeln. Das Blutgefäß 1203 dient dabei als eine Messstrecke und ist somit als ein verteiltes Element und nicht mehr als ein konzentriertes Element zu sehen. Bevorzugt wird die Messung der Verlustgrößen auf der Basis einer Zweitormessung durchgeführt. Dabei können insbesondere bei einer Ankopplung der Erfassungsvorrichtung an ein Handgelenk Primärmoden in dem dielektrischen Wellenleiter gemäß Fig. 12B angeregt werden, so dass eine Anregung von Filmwellenleitermoden in dem Filmwellenleiter gemäß Fig. 12C vermieden wird, so dass die Erfassung des Blutbildparameters genauer durchgeführt werden kann.

Um Primärmoden im dielektrischen Wellenleitersystem anzuregen, kann berücksichtigt werden, dass je nach gewählter Frequenz eines Sendesignals unterschiedliche Moden dominant sein können. Bevorzugt sind Modentypen, die eine Konzentration der Felder in der Vene 1203 aufweisen, solchen Moden vorzuziehen, bei denen die Felder in der Hautschicht 1201 konzentriert sind. Aufgrund der dielektrischen Eigenschaften des in Fig. 12B dargestellten dielektrischen Wellenleiters zeigt sich, dass für bestimmte Modentypen longitudinale Komponenten $E_{longitudinal}$, $H_{longitudinal}$ in Ausbreitungsrichtung, d. h. in Richtung eines Venenverlaufs, stärker als

die transversalen Komponenten $E_{transversal}$, $H_{transversal}$, d. h. quer zum Venenverlauf, sind. Bevorzugt werden daher in dem zu erfassenden Frequenzbereich diejenigen Moden angeregt, welche eine maximale Einkopplung der Mikrowellenleistung in das Blutgefäß 1203 ermöglichen.

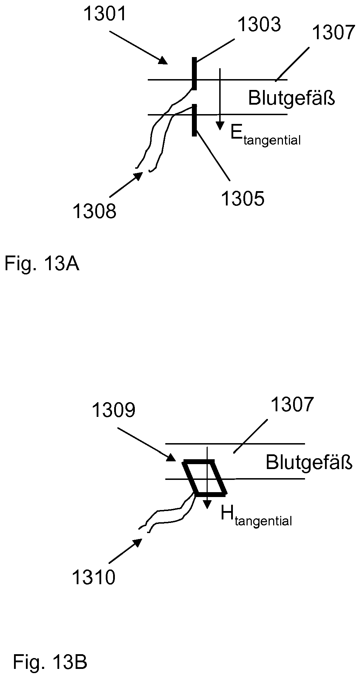

In den Figuren 13A bis 13D sind beispielhaft einige Antennen dargestellt, welche als Sendeantennen, d. h. Erreger, oder auch als Empfangsantennen eingesetzt werden können.

Die in Fig. 13A dargestellte Antenne 1301 ist als ein elektrischer Dipol mit einem ersten Antennenabschnitt 1303 und einem zweiten Antennenabschnitt 1305 ausgeführt Die Antennenabschnitte 1303 und 1305 sind voneinander beabstandet und beispielsweise quer zu einem Verlaufeines Blutgefäßes 1307 angeordnet. Eine Anregung der Antenne 1301 kann durch Zuleitungen 1308 erfolgen. Ein derart angeordneter elektrischer Dipol kann beispielsweise ein elektrisches Feld $E_{tangential}$ erzeugen, das quer zum Verlauf des Blutgefäßes bzw. zur Blutflussrichtung zeigt.

In Fig. 13B ist eine Antenne 1309 dargestellt, welche eine Rahmenantenne sein kann. Die Rahmenantenne kann beispielsweise eine Viereckform oder eine runde Form aufweisen. Bei der in Fig. 13B dargestellten Anordnung der Rahmenantenne 1309 bezüglich des Blutgefäßes 1307 wird beispielsweise ein magnetisches Feld $H_{tangential}$ angeregt, das quer zum Verlauf des Blutgefäßes 1307 bzw. quer zur Blutflussrichtung zeigt. Eine Anregung der Antenne 1309 kann durch Zuleitungen 1310 erfolgen.

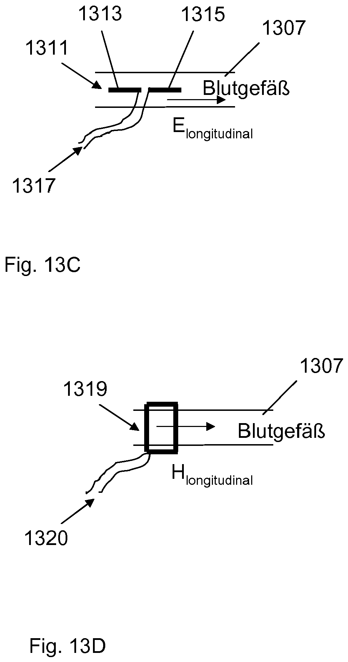

In Fig. 13C ist eine Antenne 1311 dargestellt, welche einen elektrischen Dipol mit einem ersten Antennenabschnitt 1313 und einem zweiten Antennenabschnitt 1315 bildet. Die Antennenabschnitte 1313 und 1315 sind voneinander beabstandet und werden mittels der in Fig. 13C dargestellten Zuleitungen 1317 angeregt. Der durch die Antenne 1311 gebildete elektrische Dipol ist nun derart bezüglich des Verlaufs des Blutgefäßes 1307 angeordnet, dass die Abschnitte 1313 und 1315 parallel zum Verlauf des Blutgefäßes 1307 angeordnet sind. Dadurch wird ein in Richtung des Verlaufs des Blutgefäßes weisendes elektrisches Feld mit der Feldkomponente $E_{longitudinal}$ angeregt.

Fig. 13D zeigt eine Rahmenantenne 1319, welche beispielsweise in der Gestalt eines viereckigen oder runden Rahmens, welcher eine Schleifenantenne bildet, beispielsweise als eine Patch-Antenne, gebildet sein kann. Die Rahmenantenne 1319 wird mittels Zuleitungen 1320 angeregt und ist wie es in Fig. 13D dargestellt ist, bezüglich des Verlaufs des Blutgefäßes 1307 bzw. bezüglich der Blutflussrichtung derart angeordnet, dass das magnetische Feld eine in Richtung des Verlaufs des Blutgefäßes 1307 weisende Komponente $H_{longitudinal}$ aufweist.

Der jeweils zu messende Frequenzbereich richtet sich beispielsweise danach, welche Spektrallinien, d. h. welche Absorptionslinien, zu erfassen sind. Es können beispielsweise die charakteristischen Absorptionslinien eines Stoffes oder auch ein Effekt beobachtet werden, den ein bestimmter Blutbestandteil auf die Absorptionslinien von Wasser bzw. von einer Wasserlösung mit einer Konzentration des Blutbestandteils aufweist.

Bei den in den Figuren 13A bis 13D dargestellten Antennen handelt es sich entweder um elektrische Dipole oder um magnetische Rahmenantennen. Darüber hinaus können auch Patch-Antennen eingesetzt werden. Elektrische Dipole erzeugen dominant ein elektrisches Feld in der Achse des elektrischen Dipols. Diese Achse kann entweder, wie es in Fig. 13A dargestellt ist, tangential zum Blutgefäß 1307 bzw. zur Blutflussrichtung, oder wie es in Fig. 13C dargestellt ist, in Richtung des Blutgefäßes 1307 bzw. in Blutflussrichtung ausgerichtet sein. Falls primär ein magnetisches Feld erzeugt werden soll, so kann eine Rahmenantenne als Erreger eingesetzt werden. Ist ein Flächenvektor auf der von dem die Rahmenantenne bildenden Rahmen aufgespannten Fläche quer zu dem Blutgefäß 1307 bzw. quer zur Blutflussrichtung ausgerichtet, so ist auch das magnetische Feld quer zu dem Blutgefäß 1307 ausgerichtet, wie es in Fig. 13B dargestellt ist. Zeigt der Flächenvektor hingegen in Richtung des Blutgefäßes 1307, so ist auch das Magnetfeld in Richtung des Blutgefäßes 1307 ausgerichtet, wie es beispielsweise in Fig. 13B dargestellt ist. Aus der Wahl eines der in den Figuren 13A bis 13D dargestellten Erregers ergibt sich dann beispielsweise die dominant angeregte Mode bzw. Wellentyp.

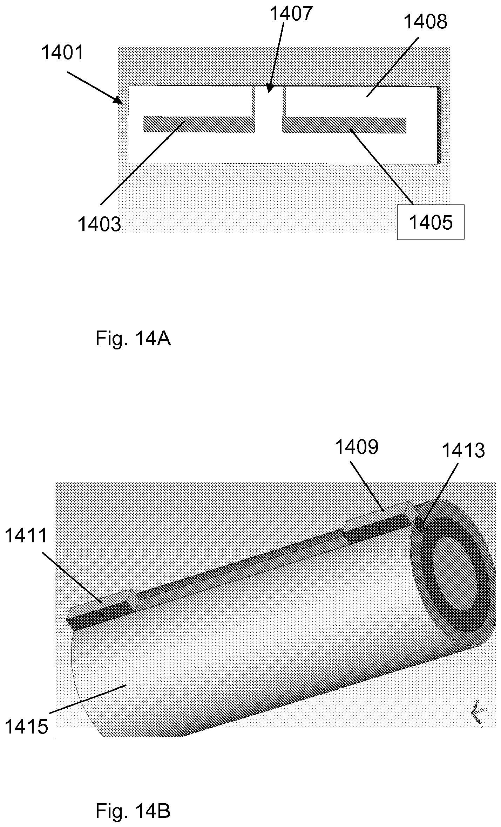

Fig. 14A zeigt eine elektrische Dipolantenne 1401, welche als eine Sendeantenne oder als eine Empfangsantenne eingesetzt werden kann. Die elektrische Dipolantenne 1401 umfasst Dipolantennenabschnitte 1403 und 1405, welche in einem Substrat 1408 angeordnet sind und mittels Zuleitungen 1407 angeregt werden können. Die Dipolantenne 1401 kann als Sendeantenne oder als Empfangsantenne eingesetzt werden.

Fig. 14B zeigt eine Erregeranordnung einer Sendeantenne 1409 eines Senders und einer Empfangsantenne 1411 eines Empfängers in Richtung eines Verlaufs eines Blutgefäßes 1413 unterhalb einer Hautschicht 1415. Die Sendeantenne 1409 und die Empfangsantenne 1411 sind beispielsweise elektrische Dipolantennen gemäß Fig. 14A. Bei der in Fig. 14B dargestellten Anordnung wird ein elektrisches Feld mit einer Feldkomponente in Richtung des Verlaufs des Blutgefäßes 1413, bzw. in Blutflussrichtung erzeugt.

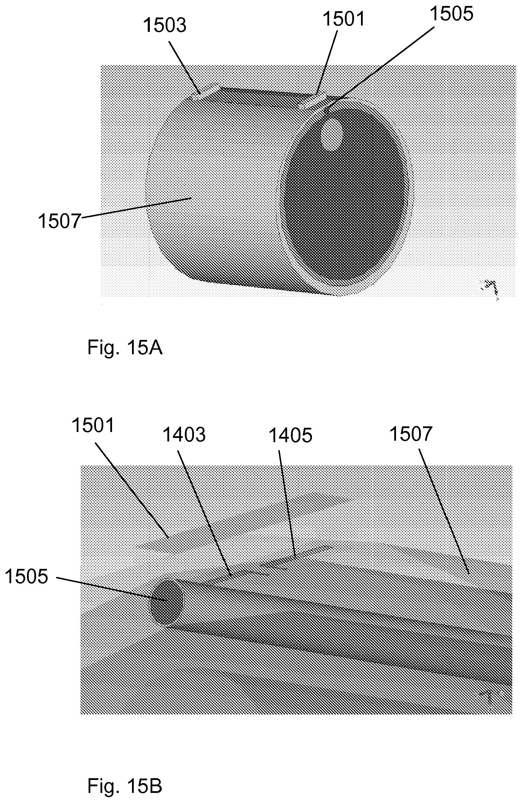

Fig. 15A zeigt eine Erregeranordnung umfassend eine Sendeantenne 1501 eines Senders und eine Empfangsantenne 1503 eines Empfängers quer zur Ausbreitungsrichtung eines Blutgefäßes 1505, d. h. quer zur Blutflussrichtung, das unter einer Hautschicht 1507 liegt. Die Sendeantenne 1501 und die Empfangsantenne 1503 können jeweils beispielsweise durch die in Fig. 14A dargestellte elektrische Dipolantenne gebildet sein. In Fig. 15B ist die Anordnung der Dipolantennenabschnitte 1403 und 1405 bezüglich der Blutflussrichtung genauer dargestellt.

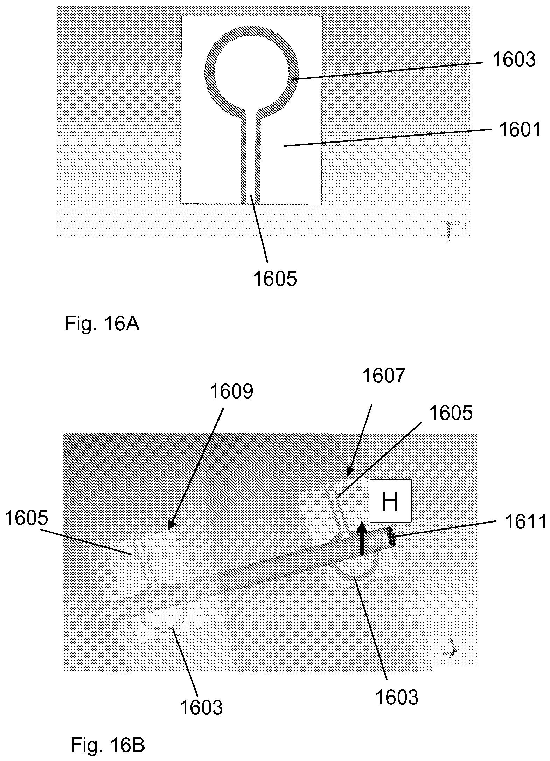

Fig. 16A zeigt eine Schleifenantenne 1601 mit einem kreisförmigen Rahmen 1603 und Zuleitungen 1605 zum Anregen des kreisförmigen Rahmens 1603. Die Schleifenantenne 1601 kann beispielsweise als eine Sendeantenne oder als eine Empfangsantenne eingesetzt werden. Der kreisförmigen Rahmen 1603 sowie die Zuleitungen 1605 können in einem Substrat angeordnet werden.

Fig. 16B zeigt eine Erregeranordnung mit einer Sendeantenne 1607 eines Senders und einer Empfangsantenne 1609 eines Empfängers, welche als Schleifenantennen gemäß Fig. 16A gebildet sein können. Die Schleifenantennen 1607, 1609 sind beispielsweise derart angeordnet, dass die kreisförmigen Rahmen 1603 oberhalb eines Blutgefäßes 1611 angeordnet sind, wobei die Zuleitungen 1605 quer zum Verlauf des Blutgefäßes 1611, d. h. quer zur Blutflussrichtung, zeigen. Dadurch wird senderseitig ein Magnetfeld H mit einer quer zum Verlauf des Blutgefäßes 1611 weisenden Komponente des Magnetfeldes erzeugt.

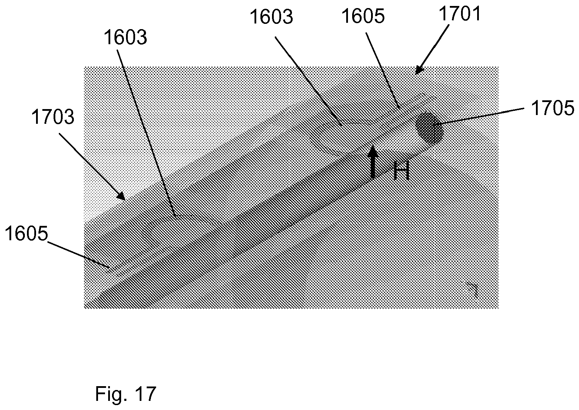

Fig. 17 zeigt eine Erregeranordnung einer Sendeantenne 1701 eines Senders und einer Empfangantenne 1703 eines Empfängers bezüglich eines Blutgefäßes 1705. Die Sendeantenne 1701 und die Empfangsantenne 1703 können beispielsweise Schleifenantennen mit der in Fig. 16A dargestellten Form sein. Sie sind beispielsweise derart angeordnet, dass die kreisrunden Rahmen 1603 jeweils oberhalb des Blutgefäßes 1705 angeordnet sind und dass die Zuleitungen 1605 voneinander weisend parallel zum Verlauf des Blutgefäßes 1705 verlaufen. Dadurch wird eine senkrecht zum Verlauf des Blutgefäßes 1705 weisende Feldkomponente H erzeugt, welche in Richtung einer Normalen der durch den kreisrunden Rahmen 1603 aufgespannten Fläche zeigt.

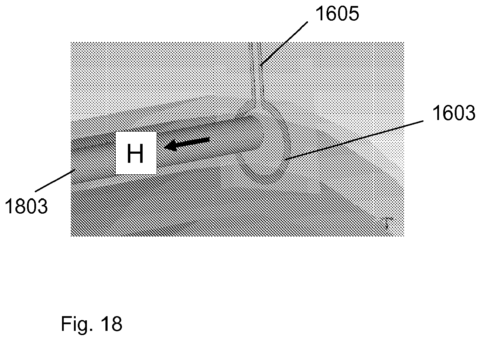

Fig. 18 zeigt eine Erregeranordnung mit einer Sendeantenne 1801 eines Senders, welche beispielsweise die in Fig. 16A dargestellte Form einer Schleifenantenne aufweist. Die Sendeantenne 1801 ist bezüglich eines Blutgefäßes 1803 beispielsweise derart angeordnet, dass eine Normale der durch den Rahmen 1603 aufgespannten Fläche in Richtung des Verlaufes des Blutgefäßes 1803 zeigt. Eine derartige Anordnung kann beispielsweise bei einem Knick des Blutgefäßes 1803 realisiert werden. Dadurch wird eine in Richtung des Verlaufes des Blutgefäßes 1803 weisende magnetische Feldkomponente H erzeugt.

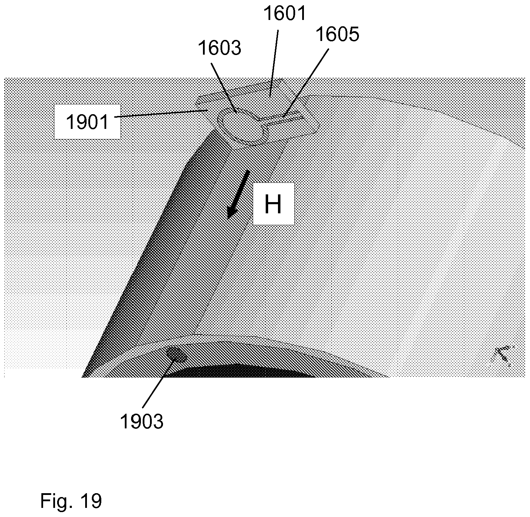

Fig. 19 zeigt eine Erregeranordnung mit einer Sendeantenne 1601, welche beispielsweise eine Schleifenantenne mit der in Fig. 16A dargestellten Form ist und in einem Substrat 1901, beispielsweise Kunststoffsubstrat, angeordnet sein kann. Die Sendeantenne 1601 ist oberhalb eines Blutgefäßes 1903 derart angeordnet, dass eine Normale der durch den kreisförmigen Rahmen 1603 aufgespannten Fläche in Richtung des Verlaufes des Blutgefäßes 1903 zeigt. Dadurch wird ein magnetisches Feld mit einer in Richtung des Verlaufes des Blutgefäßes 1903, d. h. in Blutflussrichtung, weisenden Feldkomponente H erzeugt.

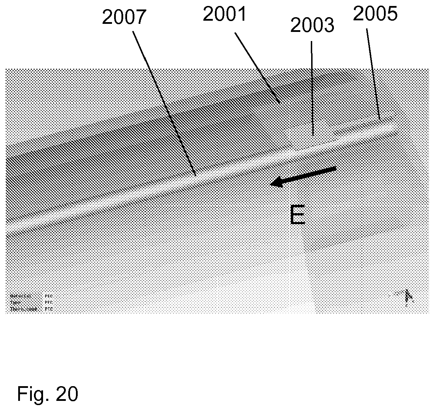

Fig. 20 zeigt eine Erregeranordnung mit einer Sendeantenne 2001, welche eine Patch- Antenne mit einer Patch-Antennenfläche 2003 und Zuleitungen 2005 sein kann. Die Patch-Antennenfläche 2003 ist beispielsweise oberhalb eines Blutgefäßes 2007 angeordnet, wodurch ein elektrisches Feld mit einer in Richtung eines Verlaufs des Blutgefäßes 2007, d. h. in Blutflussrichtung, weisenden elektrischen Feldkomponente E erzeugt wird.

Gemäß einer Ausführungsform ist der Verlustdetektor 1107 ausgebildet, beispielsweise eine skalare oder eine vektorielle Messung oder eine Leistungsmessung durchzuführen. Zur Bestimmung der Verlustgrößen kann eine einfache spektroskopische Messung durchgeführt werden, bei der der Betrag des Messparameters $S_{21}$ erfasst wird.

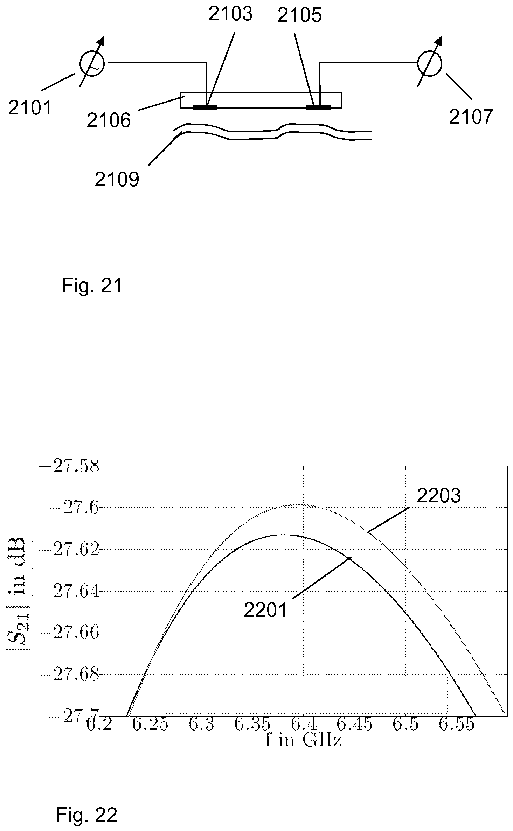

Die Messung von $|S_{21}|$ kann beispielsweise mittels der in Fig. 21 dargestellten Erfassungsvorrichtung durchgeführt werden. Die Erfassungsvorrichtung umfasst einen Sender mit einem Sendesignalgenerator 2101, welcher ein durchstimmbarer Oszillator sein kann. Ein Ausgang des Sendesignalgenerators 2101 ist mit einer Sendeantenne 2103 verbunden. Die Erfassungsvorrichtung umfasst ferner einen Empfänger mit einer Empfangsantenne 2105, deren Ausgang mit einem Verlustdetektor 2107 verbunden ist. Der Verlustdetektor kann beispielsweise einen Leistungsdetektor umfassen. Wie in Fig.21 dargestellt ist, sind die Sendeantenne 2103 und die Empfangsantenne 2105 oberhalb eines Blutgefäßes 2109 angeordnet. Der Sender kann dem Sender 1101, der Empfänger kann dem Empfänger 1105 und der Verlustdetektor 2107 kann dem Verlustdetektor 1107 entsprechen.

Durch eine weitere Messung eines Betrags des Messparameters von $S_{11}$ kann jedoch die Genauigkeit bei der Bestimmung der Verlustgrößen, d. h. der Verluste in dem Wellenleiter, noch weiter gesteigert werden. Die Verlustgrößen können beispielsweise auf der Basis der folgenden Formel bestimmt werden:

$$P_{Verlust} = 1 - |S_{11}|^2 - |S_{21}|^2,$$

wobei $P_{Verlust}$ die jeweilige Verlustgröße bezeichnet, und wobei $S_{11}$ den Eingangsreflexionsfaktor und $S_{21}$ den Vorwärtstransmissionsfaktor bezeichnen.

Zum Erfassen des Blutbildparameters, beispielsweise einer Konzentration von Blutzucker, können beispielsweise Frequenzverschiebungen von Absorptionslinien einer Wasserlösung mit Zucker untersucht werden. Fig. 22 zeigt beispielsweise eine Frequenzverschiebung eines Absorptionsmaximums 2201 bei einer ersten Blutzuckerkonzentration im Vergleich zu einer Frequenzverschiebung eines Absorptionsmaximums 2203 bei einer zweiten Blutzuckerkonzentration, welche höher als die erste Blutzuckerkonzentration. Dabei wurde als Verlustgröße beispielhaft eine Transmission um 6 GHz erfasst.

Die Frequenzverschiebung des Absorptionsmaximums kann als ein Maß für einen Blutbildparameter, beispielsweise für einen Blutzuckerspiegel, aufgefasst werden. Durch eine Beobachtung von Frequenzverschiebungen bei mehreren Absorptionen einer Wasserlösung mit Zucker kann die Messzuverlässigkeit noch weiter erhöht werden.

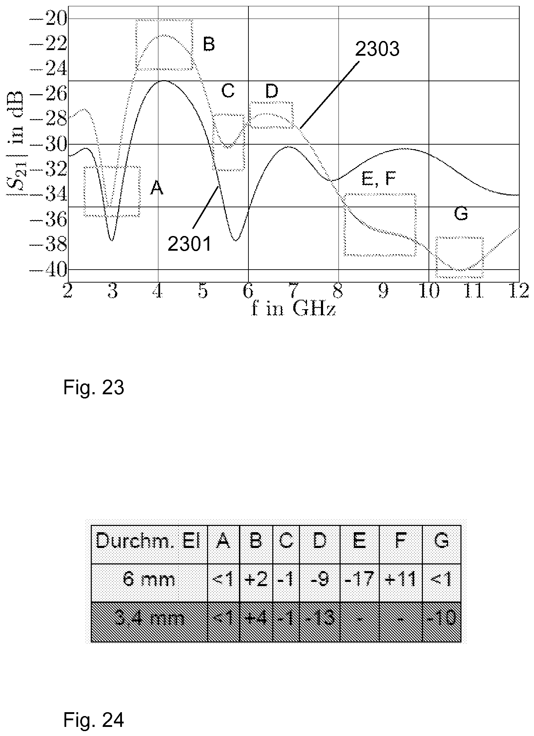

Fig. 23 zeigt beispielhaft ein breitbandiges Transmissionsverhalten von venösem Blut in einem Handgelenk. Dabei verdeutlichen die Verläufe 2301 und 2303 unterschiedliche Frequenzlagen von Absorptionslinien bei unterschiedlichen Blutzuckerkonzentrationen.Zur Erfassung des Blutbildparameters, wie beispielsweise der Konzentration des Blutzuckers, können beispielsweise gezielt Frequenzverschiebungen der Absorptionen A, B, C, D, E, F und G erfasst werden. So kann beispielsweise für jede Frequenz eines Absorptionsmaximums und/oder eines Absorptionsminimums eine Verschiebung in Richtung höherer oder niedrigerer Frequenzen je nach Blutzuckerspiegel beispielsweise in einem Frequenzbereich zwischen 2 GHz und 12 GHz beobachtet werden.

Fig. 24 zeigt beispielhaft Frequenzverschiebungen der in Fig. 23 dargestellten Absorptionen A, B, C, D, E, F und G für ein Blutgefäß mit 6 mm Durchmesser und für ein Blutgefäß mit 3,4 mm Durchmesser. Zu erkennen ist, dass die Absorptionen für eine Zuckerspiegelvariation Frequenzverschiebungen sowohl in positiver als auch in negativer Richtung aufweisen können. Die Erfassung von mehreren Absorptionen bzw. Absorptionslinien ermöglicht somit eine genauere Erfassung eines Blutbildparameters, beispielsweise des Blutzuckerspiegels.

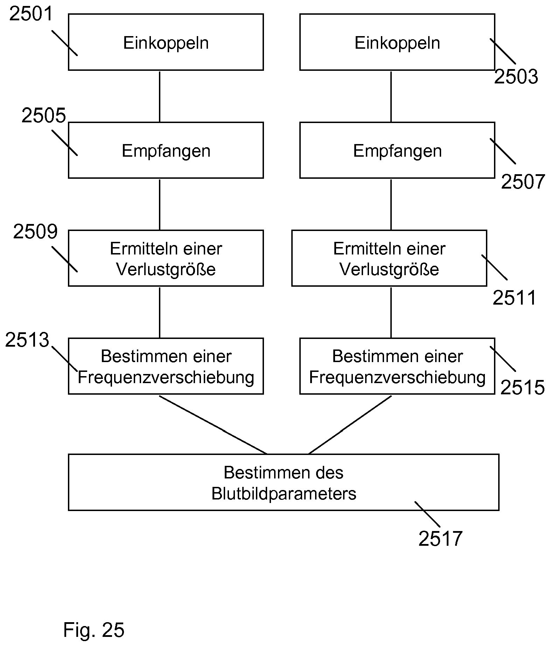

Fig. 25 zeigt ein Diagramm eines Verfahrens zum Erfassen eines Blutbildparameters von Blut in einem Blutgefäß. Das Verfahren umfasst das Einkoppeln 2501 eines ersten Sendesignals mit einer ersten Frequenz in das Blutgefäß, das Einkoppeln 2503 eines zweiten Sendesignals mit einer zweiten Frequenz in das Blutgefäß, das Empfangen 2505 eines ersten Empfangssignals bei der ersten Frequenz, das Empfangen 2507 eines zweiten Empfangssignals bei der zweiten Frequenz, das Ermitteln 2509 einer ersten Verlustgröße auf der Basis des ersten Sendesignals und des ersten Empfangssignals bei der ersten Frequenz, das Ermitteln 2511 einer zweiten Verlustgröße auf der Basis des zweiten Sendesignals und des zweiten Empfangssignals bei der zweiten Frequenz, das Bestimmen 2513 einer ersten Frequenzverschiebung der ersten Verlustgröße relativ zu einer ersten Referenzverlustgröße, das Bestimmen 2515 einer zweiten Frequenzverschiebung der zweiten Verlustgröße relativ zu einer zweiten Referenzverlustgröße, und das Bestimmen 2517 des Blutbildparameters auf der Basis der ersten Frequenzverschiebung und der zweiten Frequenzverschiebung.

Das in Fig. 25 dargestellte Verfahren kann beispielsweise durch die in Fig. 11 dargestellte Erfassungsvorrichtung ausgeführt werden.

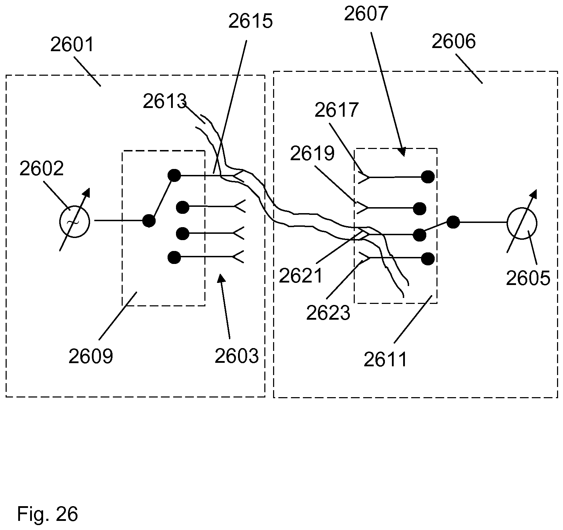

Fig. 26 zeigt eine Erfassungsvorrichtung mit einem Sender 2601, der beispielsweise einen durchstimmbaren Oszillator 2602 sowie eine Mehrzahl von Sendeantennen 2603 aufweist. Die Erfassungsvorrichtung umfasst ferner einen Verlustdetektor 2605, welcher beispielsweise einen Leistungsdetektor aufweisen kann. Ferner ist ein Empfänger 2606 mit einer Mehrzahl von Empfangsantennen 2607 vorgesehen.

Ein Ausgang des durchstimmbaren Oszillators 2602 ist beispielsweise mittels einer Schaltmatrix 2609 mit jedem Antenneneingang beispielsweise der Reihe nach oder in beliebiger Reihenfolge schaltbar verbindbar. In Analogie hierzu ist jeder Ausgang einer Empfangsantenne der Mehrzahl der Empfangsantennen 2607 mittels einer Schaltmatrix 2611 mit dem Verlustdetektor 2605 verbindbar.

Mittels der Schaltmatrix 2611 sowie der Schaltmatrix 2609 kann beispielsweise dasjenige Paar umfassend eine Sendeantenne und eine Empfangsantenne ausgewählt werden, das die beste Einkopplung eines Mikrowellensignals in ein in Fig. 26 schematisch dargestelltes Blutgefäß 2613 ermöglicht. Mittels der Schaltmatrizen 2609 und 2611 werden die Antennenpaare der Reihe nach ausgewählt beginnend beispielsweise mit einer ersten Sendeantenne 2615, mittels welcher ein Sendesignal ausgesendet wird. Die Schaltmatrizen 2609, 2611 können Schalter, beispielsweise Transistoren, aufweisen.

Empfangsseitig werden mittels der Schaltmatrix 2611 der Reihe nach dieEmpfangsantennen beginnend beispielsweise mit der Empfangsantenne 2617 zum Empfang eines entsprechenden Empfangssignals ausgewählt, wobei auf der Basis des Sendesignals und des Empfangssignals eine Verlustgröße erfasst wird. Im nächsten Schritt wird beispielsweise die Empfangsantenne 2619 ausgewählt, wobei wiederum auf der Basis des Sendesignals und eines durch die Empfangsantenne 2619 empfangenen Empfangssignals eine Verlustgröße mittels des Verlustdetektors erfasst wird. Danach wird beispielsweise die Empfangsantenne 2621 ausgewählt, wobei auf der Basis des Sendesignals und eines Empfangssignals eine weitere Verlustgröße erfasst wird. Im nächsten Schritt wird die Empfangsantenne 2623 ausgewählt und es wird auf der Basis des Sendesignals sowie eines durch die Empfangsantenne 2623 empfangenenEmpfangssignals eine weitere Verlustgröße bestimmt. Im nächsten Schritt kann die Schaltmatrix 2609 beispielsweise eine weitere Sendeantenne auswählen, wobei die vorgenannten Schritte wiederholt werden können. Durch einen Vergleich der ermittelten Verlustgrößen wird beispielsweise die kleinste Verlustgröße ausgewählt. In dem in Fig. 26 dargestellten Beispiel ist beispielsweise zu erwarten, dass die Erfassungskonfiguration mit der Sendeantenne 2615 und der Empfangsantenne 2621 mit den geringsten Einkoppelverlusten behaftet ist, weil die Antennen 2615, 2621 unmittelbar oberhalb des Blutgefäßes liegen und somit eine optimale Einkopplung eines Signals in das Blutgefäß 2613 ermöglichen. Die ausgewählte Erfassungskonfiguration kann beispielsweise zur Erfassung eines Blutbildparameters herangezogen werden. Die vorstehend beschriebenen Auswahlschritte können in beliebiger Reihenfolge durchgeführt werden. So können beispielsweise für die Sendeantenne 2615 alle oder einige der Empfangsantennen 2607 getestet werden.

Die Sendeantennen 2603 bzw. die Empfangsantennen 2607 können sich hinsichtlich ihres Ortes und/oder hinsichtlich ihrer Feldkomponente, welche dominant angeregt werden soll, unterscheiden. Durch die Schaltmatrizen 2609 und 2611 ist dabei gewährleistet, dass für die jeweils gewählte Frequenz der optimale Erregertyp, beispielsweise Schleifenantenne, elektrische Dipolantenne, Patch-Antenne, oder Erregerort ausgewählt werden kann.

Die in Fig. 26 dargestellte Erfassungsvorrichtung kann beispielsweise in einem aufpumpbaren Armband integriert sein. Zwischen den Erfassungen der Verlustgrößen, welche beispielsweise durch Messungen der Steuerparameter erfolgen können, kann Luft aus dem Armband abgelassen werden, so dass die Haut belüftet wird und sich kein Schweiß bildet. Ein Zeitabstand zwischen den Messungen kann dabei variabel erfolgen. Messungen können beispielsweise in Abständen von 10 Minuten durchgeführt werden. Je nach Bedarf können jedoch häufigere Messungen durchgeführt werden, wobei die Häufigkeit der Messungen beispielsweise durch die Zeitpunkte der Einnahme der Mahlzeiten bestimmt sein kann.

Da insbesondere in den Pausen zwischen den Messungen die auf der Haut liegenden Sende- bzw. Empfangsantennen, welche jeweils durch eine Elektrodenplatte gebildet sein können, verrutschen können, kann durch die in Fig. 26 dargestellte Auswahl mehrerer Erreger sichergestellt werden, dass ein Erreger ausgewählt wird, welcher über dem Blutgefäß 2613 liegt. Mittels der jeweiligen Umschaltmatrix 2609 und 2611 kann somit derjenige Erreger ausgewählt werden, welcher ein Maximum einer Einkopplung von Mikrowellenenergie in das Blutgefäß 2613 ermöglicht.

**Patentansprüche**

1. Armband (100, 200, 300), mit:

   einer Erfassungsvorrichtung (101, 201, 301) zum Erfassen eines Blutbildparameters von Blut in einem Blutgefäß des Armes, wobei die Erfassungsvorrichtung (101, 201, 301) einen Sender hat, welcher wiederum aufweist:

   - eine Anzahl von Sendeantennen zum Aussenden von zumindest einem Sendesignal,
   - einen Empfänger mit einer Anzahl von Empfangsantennen zum Empfangen von zumindest einem Empfangssignal,
- einen Prozessor, welcher konfiguriert ist, eine erste Erfassungskonfiguration, umfassend eine Sendeantenne der Anzahl der Sendeantennen und eine Empfangsantenne der Anzahl der Empfangsantennen, auszuwählen, und eine zweite Erfassungskonfiguration, umfassend eine Sendeantenne der Anzahl der Sendeantennen und eine Empfangsantenne der Anzahl von Empfangsantennen, auszuwählen,
- einen Verlustdetektor, welcher konfiguriert ist, bei Auswahl der ersten Erfassungskonfiguration zum Aussenden eines Sendesignals eine erste Verlustgröße auf der Basis des Sendesignals und des Empfangssignals zu erfassen, und bei Auswahl der zweiten Erfassungskonfiguration zum Aussenden eines Sendesignals eine zweite Verlustgröße auf der Basis des Sendesignals und des Empfangssignals zu erfassen, wobei der Prozessor konfiguriert ist, eine Erfassungskonfiguration unter Vergleich der erfassten ersten Verlustgröße und der erfassten zweiten Verlustgröße zum Erfassen des Blutbildparameters auszuwählen,

und

einer Einstellvorrichtung (103, 203, 303) zum Einstellen eines vorgegebenen Anpressdruckes des Armbandes auf den Arm, wobei das Armband (100, 200, 300) als ein aufblasbares Armband ausgebildet ist.

2. Armband nach Anspruch 1, wobei die Einstellvorrichtung (103, 203, 303) ausgebildet ist, den Anpressdruck des Armbandes zumindest während des Erfassens des Blutbildparameters durch die Erfassungsvorrichtung (101, 201, 301) auf den vorgegebenen Anpressdruck einzustellen.

3. Armband nach Anspruch 1 oder 2, wobei die Einstellvorrichtung (203, 303) eine Sensoreinrichtung (205, 305) zum Messen eines aktuellen Anpressdruckes des Armbandes (200, 300) auf den Arm und eine Steuereinrichtung (207, 307) zum Einstellen des vorgegebenen Anpressdruckes in Abhängigkeit des gemessenen aktuellen Anpressdruckes aufweist.

4. Armband nach Anspruch 3, wobei die Sensoreinrichtung (205, 305) auf der Innenseite (209, 309) des Armbandes (200, 300) angeordnet ist.

5. Armband nach einem der vorstehenden Ansprüche, wobei die Einstellvorrichtung (303) eine Luftpumpe (311) aufweist, welche ausgebildet ist, das Armband (300) zum Einstellen des vorgegebenen Anpressdruckes aufzublasen.

6. Armband nach einem der vorstehenden Ansprüche, wobei die Einstellvorrichtung (303) eine Sensoreinrichtung (305) zum Messen eines aktuellen Anpressdruckes des Armbandes (300) auf den Arm, eine Steuereinrichtung (307) zum Bereitstellen eines Steuersignals in Abhängigkeit des gemessenen aktuellen Anpressdruckes und eine mittels des bereitgestellten Steuersignals gesteuerte Luftpumpe (311) zum Aufblasen des Armbandes (300) umfasst.

7. Armband nach einem der vorstehenden Ansprüche, wobei die Erfassungsvorrichtung (401) Elektroden (403, 405) umfasst, welche zur Einkopplung zumindest eines Hochfrequenzsignals in das Blutgefäß eingerichtet sind.

8. Armband nach Anspruch 7, wobei die Einstellvorrichtung (103, 203, 303) ausgebildet ist, den Anpressdruck der Elektroden (403, 405) auf den Arm auf den vorgegebenen Anpressdruck einzustellen.

9. Armband nach einem der vorstehenden Ansprüche, wobei die Einstellvorrichtung (103, 203, 303) ausgebildet ist, die Anpresskräfte des Armbandes (100, 200, 300) während des Erfassens des Blutbildparameters durch die Erfassungsvorrichtung (101, 201, 301) gleichmäßig auf den Arm zu verteilen.

10. Armband nach einem der vorstehenden Ansprüche, wobei die Einstellvorrichtung (103, 203, 303) ausgebildet ist, ein gleichmäßiges Anliegen des Armbandes (100, 200, 300) während des Erfassens des Blutbildparameters durch die Erfassungsvorrichtung (101, 201, 301) bereitzustellen.

11. Armband nach einem der vorstehenden Ansprüche, wobei die Einstellvorrichtung (103, 203, 303) ausgebildet ist, den Anpressdruck des Armbandes (100, 200, 300) während des Erfassens des Blutbildparameters durch die Erfassungsvorrichtung (101, 201, 301) konstant zu halten.

12. Armband nach einem der vorstehenden Ansprüche, welches ferner einen Speicher zum Bereitstellen des vorgegebenen Anpressdruckes umfasst.

13. Armband nach einem der vorstehenden Ansprüche, wobei der zumindest eine Blutbildparameter eine Glucosekonzentration in dem Blut, eine Laktatkonzentration in dem Blut, eine Laktatkonzentration in dem Blut oder eine Sauerstoffkonzentration in dem Blut umfasst.

**Claims**

1. A bracelet (100, 200, 300), with:

    a detecting apparatus (101, 201, 301) for detecting a blood count parameter of blood in a blood vessel of the arm,
    said detecting apparatus (101, 201, 301) having a transmitter, which in turn comprises:

        - a number of transmission antennas for transmitting at least one transmission signal,
        - a receiver having a number of reception antennas for receiving at least one reception signal,
        - a processor configured to select:
        a first detection configuration comprising a transmission antenna of the number of transmission antennas and a reception antenna of the number of reception antennas, and a second detection configuration comprising a transmission antenna of the number of transmission antennas and a reception antenna of the number of reception antennas,
        - when the first detection configuration is selected,
        a loss detector configured for transmitting a transmission signal in order to detect a first loss quantity on the basis of the transmission signal and the reception signal,
        and when the second detection configuration is selected, for transmitting a transmission signal in order to detect
        a second loss quantity on the basis of the transmission signal and the reception signal,
        wherein the processor is configured for selecting a detection configuration by comparing the detected first loss quantity and the detected second loss quantity to detect the blood count parameter,

    and
    an adjusting apparatus (103, 203, 303) for adjusting a predetermined contact pressure of the bracelet on the arm, the bracelet (100, 200, 300) being designed as an inflatable bracelet.

2. The bracelet according to claim 1, wherein the adjusting apparatus (103, 203, 303) is designed to adjust the contact pressure of the bracelet to the predetermined contact pressure at least during the detection of the blood count parameter by the detection apparatus (101, 201, 301).

3. The bracelet according to claim 1 or 2, wherein the adjusting apparatus (203, 303) comprises a sensor device (205, 305) for measuring a current contact pressure of the bracelet (200, 300) on the arm and a control device (207, 307) for adjusting the predetermined contact pressure depending on the measured current contact pressure.

4. The bracelet according to claim 3, wherein the sensor device (205, 305) is arranged on the inside (209, 309) of the bracelet (200, 300).

5. The bracelet according to anyone of the preceding claims, wherein the adjusting apparatus (303) comprises an air pump (311) designed to inflate the bracelet (300) for adjusting the predetermined contact pressure.

6. The bracelet according to anyone of the preceding claims, wherein the adjusting apparatus (303) comprises:

    a sensor device (305) for measuring a current contact pressure of the bracelet (300) on the arm,
    a control device (307) for providing a control signal depending on the measured current contact pressure and an air pump (311) controlled by means of the provided control signal for inflating the bracelet (300).

7. The bracelet according to anyone of the preceding claims, wherein the detecting apparatus (401) comprises electrodes (403, 405) which are set up for coupling at least one high-frequency signal into the blood vessel.

8. The bracelet according to claim 7, wherein the adjusting apparatus (103, 203, 303) is designed to adjust the contact pressure of the electrodes (403, 405) on the arm to the predetermined contact pressure.

9. The bracelet according to anyone of the preceding claims, wherein the adjusting apparatus (103, 203, 303) is designed to evenly spread the contact forces of the bracelet (100, 200, 300) over the arm during the detection of the blood count parameter by the detecting apparatus (101, 201, 301).

10. The bracelet according to anyone of the preceding claims, wherein the adjusting apparatus (103, 203, 303) is designed to provide an evenly abutting of the bracelet (100, 200, 300) during the detection of the blood count parameter by the detecting apparatus (101, 201, 301).

11. The bracelet according to anyone of the preceding claims, wherein the adjusting apparatus (103, 203, 303) is designed to maintain the contact pressure of the bracelet (100, 200, 300) constant during the detection of the blood count parameter by the detecting apparatus (101, 201, 301).

**12.** The bracelet according to anyone of the preceding claims, which further comprises a memory for providing the predetermined contact pressure.

**13.** The bracelet according to anyone of the preceding claims, wherein the at least one blood count parameter comprises:

a glucose concentration in the blood,
a lactate concentration in the blood
or an oxygen concentration in the blood.

**Revendications**

**1.** Un bracelet (100, 200, 300), doté:

d'un appareil de détection (101, 201, 301) permettant de détecter un paramètre de numération globulaire du sang dans un vaisseau sanguin du bras,
ledit appareil de détection (101, 201, 301) ayant un émetteur, qui à son tour comprend:

- un certain nombre d'antennes d'émission pour émettre au moins un signal d'émission,
- un récepteur ayant un certain nombre d'antennes de réception pour recevoir au moins un signal de réception,
- un processeur, configuré pour sélectionner :

une première configuration de détection,
comprenant une antenne d'émission du certain nombre d'antennes d'émission et une antenne de réception du certain nombre d'antennes de réception,
et une seconde configuration de détection,
comprenant une antenne d'émission du certain nombre d'antennes d'émission et une antenne de réception du certain nombre d'antennes de réception.

- un détecteur de perte, configuré,
lorsque la première configuration de détection est sélectionnée,
pour émettre un signal d'émission afin de détecter une première quantité de perte sur la base du signal d'émission et du signal de réception,

et lorsque la seconde configuration de détection est sélectionnée,
pour émettre un signal d'émission afin de détecter une seconde quantité de perte sur la base du signal d'émission et du signal de réception,
où le processeur est configuré pour sélectionner une configuration de détection en comparant la première quantité de perte détectée et la seconde quantité de perte détectée afin de détecter le paramètre de numération globulaire,

et

un appareil de réglage (103, 203, 303) permettant de régler une pression de contact prédéfinie du bracelet sur le bras, le bracelet (100, 200, 300) étant conçu comme un bracelet gonflable.

**2.** Le bracelet selon la première revendication, où l'appareil de réglage (103, 203, 303) est conçu de façon à régler la pression de contact du bracelet sur la pression de contact prédéfinie au moins pendant la détection du paramètre de numération globulaire par l'appareil de détection (101, 201, 301) .

**3.** Le bracelet selon la revendication 1 ou 2, où l'appareil de réglage (203, 303) comprend un dispositif de capteur (205, 305) pour mesurer une pression de contact actuelle du bracelet (200, 300) sur le bras ainsi qu'un dispositif de commande (207, 307) pour régler la pression de contact prédéfinie en fonction de la pression de contact mesurée actuelle.

**4.** Le bracelet selon la revendication 3, où le dispositif de capteur (205, 305) est agencé sur la face intérieure (209, 309) du bracelet (200, 300).

**5.** Le bracelet selon l'une quelconque des revendications précédentes, où l'appareil de réglage (303) comprend une pompe à air (311) conçue pour gonfler le bracelet (300) afin de régler la pression de contact prédéfinie.

**6.** Le bracelet selon l'une quelconque des revendications précédentes, où l'appareil de réglage (303) comprend:

un dispositif de capteur (305) pour mesurer une pression de contact actuelle du bracelet (300) sur le bras,
un dispositif de commande (307) pour fournir un signal de commande en fonction de la pression de contact mesurée actuelle,
ainsi qu'une pompe à air (311) commandée au moyen du signal de commande fourni pour gonfler le bracelet (300).

**7.** Le bracelet selon l'une quelconque des revendications précédentes, où l'appareil de détection (401) comprend des électrodes (403, 405) installées pour coupler au moins un signal haute fréquence dans le vaisseau sanguin.

**8.** Le bracelet selon la revendication 7, où l'appareil de réglage (103, 203, 303) est conçu pour régler la pression de contact des électrodes (403, 405) sur le bras sur la base de la pression de contact prédéfinie.

**9.** Le bracelet selon l'une quelconque des revendications précédentes, où l'appareil de réglage (103, 203, 303) est conçu pour répartir uniformément les forces de contact du bracelet (100, 200, 300) sur le bras pendant la détection du paramètre de numération globulaire par l'appareil de détection (101, 201, 301).

**10.** Le bracelet selon l'une quelconque des revendications précédentes, où l'appareil de réglage (103, 203, 303) est conçu pour assurer une application uniforme du bracelet (100, 200, 300) pendant la détection du paramètre de numération globulaire par l'appareil de détection (101, 201, 301).

**11.** Le bracelet selon l'une quelconque des revendications précédentes, où l'appareil de réglage (103, 203, 303) est conçu pour maintenir de façon constante la pression de contact du bracelet (100, 200, 300) pendant la détection du paramètre de numération globulaire par l'appareil de détection (101, 201, 301).

**12.** Le bracelet selon l'une quelconque des revendications précédentes, qui comprend en outre une mémoire pour fournir la pression de contact prédéfinie.

**13.** Le bracelet selon l'une quelconque des revendications précédentes où au moins un paramètre de numération globulaire comprend :

     une concentration de glucose dans le sang,
     une concentration de lactate dans le sang
     ou une concentration d'oxygène dans le sang.

Fig. 1

Fig. 2

Fig. 3

Fig. 4

Ausstatten mit
Erfassungsvorrichtung — 501

Einstellen des
Anpressdruckes — 503

Fig. 5

Fig. 6

Fig. 7

Fig. 8

Fig. 9

```
┌─────────────────────────────┐
│  Einkoppeln eines ersten und │──── 1001
│  eines zweiten Sendesignals  │
└─────────────────────────────┘
              │
              ▼
┌─────────────────────────────┐
│  Empfangen eines ersten und  │──── 1003
│ eines zweiten Empfangssignals│
└─────────────────────────────┘
              │
              ▼
┌─────────────────────────────┐
│       Ermitteln einer        │──── 1005
│      ersten Verlustgröße     │
└─────────────────────────────┘
              │
              ▼
┌─────────────────────────────┐
│       Ermitteln einer        │──── 1007
│     zweiten Verlustgröße     │
└─────────────────────────────┘
              │
              ▼
┌─────────────────────────────┐
│       Bestimmen einer        │──── 1009
│   Relaxationszeitkonstante   │
└─────────────────────────────┘
```

Fig. 10

1103

Sender

Empfänger

1101

1105

Verlustdetektor

1107

Speicher

Prozessor

1109

1111

1100

Fig. 11

Fig. 12A

Fig. 12B

Fig. 12C

1301

1303

1307

Blutgefäß

$E_{tangential}$

1305

1308

Fig. 13A

1309

1307

Blutgefäß

$H_{tangential}$

1310

Fig. 13B

1313  1315

1311  1307

Blutgefäß

$E_{longitudinal}$

1317

Fig. 13C

1307

1319

Blutgefäß

$H_{longitudinal}$

1320

Fig. 13D

Fig. 14A

Fig. 14B

1503        1501        1505

1507

Fig. 15A

1501        1403        1405        1507

1505

Fig. 15B

1603

1601

1605

Fig. 16A

1609

1607

1605

1605

H

1611

1603

1603

Fig. 16B

Fig. 17

Fig. 18

Fig. 19

Fig. 20

Fig. 21

Fig. 22

Fig. 23

| Durchm. El | A | B | C | D | E | F | G |
|---|---|---|---|---|---|---|---|
| 6 mm | <1 | +2 | -1 | -9 | -17 | +11 | <1 |
| 3,4 mm | <1 | +4 | -1 | -13 | - | - | -10 |

Fig. 24

2501

Einkoppeln

Einkoppeln

2503

2505

Empfangen

Empfangen

2507

2509

Ermitteln einer
Verlustgröße

Ermitteln einer
Verlustgröße

2511

2513

Bestimmen einer
Frequenzverschiebung

Bestimmen einer
Frequenzverschiebung

2515

Bestimmen des
Blutbildparameters

2517

Fig. 25

Fig. 26

**IN DER BESCHREIBUNG AUFGEFÜHRTE DOKUMENTE**

**In der Beschreibung aufgeführte Patentdokumente**

- DE 102009011381 A1
- WO 2006089763 A1
- WO 2010118537 A
- US 2005256411 A1

**In der Beschreibung aufgeführte Nicht-Patentliteratur**

- **CADUFF et al.** Non-invasive glucose monitoring in patients with Type 1 diabetes: A multi-sensor system combining sensors for dielectric and optical characterization of skin. *Biosensors and Bioelectronics,* 2009, vol. 24, 2778-2784
- **JEAN et al.** A microwave frequency sensor for non-invasive blood-glucose measurement, SAS 2008. *IEEE Sensors Applications Symposium,* 12. Februar 2008
- **M. MCCLUNG.** Calibration methodology for a microwave non-invasive glucose sensor. *Master Thesis, Baylor University,* Mai 2008
- **NETTER, F.N.** Atlas der Anatomie. Thieme-Verlag, 2006